# EUROPEAN PATENT SPECIFICATION

(11) **EP 1 086 214 B1**
(45) Date of publication and mention of the grant of the patent: **25.11.2009**
(21) Application number: 99928580.2
(22) Date of filing: 10.06.1999
(51) Int. Cl.: C12N 15/00

(54) **MICRONEEDLE DEVICES AND METHODS OF THEIR MANUFACTURE**
MIKRONADEL-EINRICHTUNGEN UND VERFAHREN ZUR DEREN HERSTELLUNG
DISPOSITIFS A MICROAIGUILLES ET PROCEDES DE LEUR FABRICATION

(30) Priority: 10.06.1998 US 95221; 21.05.1999 US 316229
(43) Date of publication of application: 28.03.2001
(73) Proprietor: GEORGIA TECH RESEARCH CORPORATION, Atlanta, GA 30332-0415 (US)
(72) Inventor: PRAUSNITZ, Mark, R., Decatur, GA 30030 (US); ALLEN, Mark, G., Atlanta, GA 30328 (US); MCALLISTER, Devin, V., Holley, NY 14470 (US); HENRY, Sebastien, F-51160 Ay (FR); CROS, Florent, Paul, Marcel, Atlanta, GA 30318 (US)
(74) Representative: Stevens, Ian Edward
(86) International application number: PCT/US1999/013226
(87) International publication number: WO 1999/064580

(56) References cited:
- US-A- 5 364 374
- US-A- 5 457 041
- US-A- 5 591 139
- US-A- 5 697 901

## Description

### Background of the Invention

This invention is generally in the field of devices for the transport of therapeutic or biological molecules across tissue barriers, such as for drug delivery.

Numerous drugs and therapeutic agents have been developed in the battle against disease and illness. However, a frequent limitation of these drugs is their delivery: how to transport drugs across biological barriers in the body (e.g., the skin, the oral mucosa, the blood-brain barrier), which normally do not transport drugs at rates that are therapeutically useful or optimal.

Drugs are commonly administered orally as pills or capsules. However, many drugs cannot be effectively delivered in this manner, due to degradation in the gastrointestinal tract and/or elimination by the liver. Moreover, some drugs cannot effectively diffuse across the intestinal mucosa. Patient compliance may also be a problem, for example, in therapies requiring that pills be taken at particular intervals over a prolonged time.

Another common technique for delivering drugs across a biological barrier is the use of a needle, such as those used with standard syringes or catheters, to transport drugs across (through) the skin. While effective for this purpose, needles generally cause pain; local damage to the skin at the site of insertion; bleeding, which increases the risk of disease transmission; and a wound sufficiently large to be a site of infection. The withdrawal of bodily fluids, such as for diagnostic purposes, using a conventional needle has these same disadvantages. Needle techniques also generally require administration by one trained in its use. The needle technique also is undesirable for long term, controlled continuous drug delivery.

Similarly, current methods of sampling biological fluids are invasive and suffer from the same disadvantages. For example, needles are not preferred for frequent routine use, such as sampling of a diabetic's blood glucose or delivery of insulin, due to the vascular damage caused by repeated punctures. No alternative methodologies are currently in use. Proposed alternatives to the needle require the use of lasers or heat to create a hole in the skin, which is inconvenient, expensive, or undesirable for repeated use.

An alternative delivery technique is the transdermal patch, which usually relies on diffusion of the drug across the skin. However, this method is not useful for many drugs, due to the poor permeability (i.e effective barrier properties) of the skin. The rate of diffusion depends in part on the size and hydrophilicity of the drug molecules and the concentration gradient across the stratum corneum. Few drugs have the necessary physiochemical properties to be effectively delivered through the skin by passive diffusion. Iontophoresis, electroporation, ultrasound, and heat (so-called active systems) have been used in an attempt to improve the rate of delivery. While providing varying degrees of enhancement, these techniques are not suitable for all types of drugs, failing to provide the desired level of delivery. In some cases, they are also painful and inconvenient or impractical for continuous controlled drug delivery over a period of hours or days. Attempts have been made to design alternative devices for active transfer of drugs, or analyte to be measured, through the skin.

US 5,457,041 discloses an array of micro-needles extending from a support substrate and having tip portions shaped and dimensioned to (1) carry a biological substance and (2) pierce the surface of a target tissue and penetrate target cells within the tissue where the biological substance transfers from the tip portion and deposits within the target cells.

For example, U.S. Patent No. 5,979,326 to Godshall et al. and PCT WO 96/37256 by Silicon Microdevices, Inc. disclose a transdermal drug delivery apparatus that includes a cutter portion having a plurality of microprotrusions, which have straight sidewalls, extending from a substrate that is in communication with a drug reservoir. In operation, the microprotrusions penetrate the skin until limited by a stop region of the substrate and then are moved parallel to the skin to create incisions. Because the microprotrusions are dragged across the skin, the device creates a wound sufficiently large to be a sift of infection. Channels in the substrate adjacent to the microprotrusions allow drug from the reservoir to flow to the skin near the area disrupted by the microprotrusions. Merely creating a wound, rather than using a needle which conveys drug through an enclosed channel into the site of administration, also creates more variability in dosage.

U.S. Patent No. 5,250,023 to Lee et al. discloses a transdermal drug delivery device, which includes a plurality of skin needles having a diameter in the range of 50 to 400 µm. The skin needles are supported in a water-swellable polymer substrate through which a drug solution permeates to contact the surface of the skin. An electric current is applied to the device to open the pathways created by the skin needles, following their withdrawal from the skin upon swelling of the polymer substrate.

PCT WO 93/17754 by Gross et al. discloses another transdermal drug delivery device that includes a housing having a liquid drug reservoir and a plurality of tubular elements for transporting liquid drug into the skin. The tubular elements may be in the form of hollow needles having inner diameters of less than 1 mm and an outer diameter of 1.0 mm.

While each of these devices has potential use, there remains a need for better drug delivery devices, which make smaller incisions, deliver drug with greater efficiency (greater drug delivery per quantity applied) and less variability of drug administration, and/or are easier to use.

It is therefore an object of the present invention to provide a microneedle device for relatively painless, controlled, safe, convenient transdermal delivery of a variety of drugs.

It is another object of the present invention to provide a microneedle device for controlled sampling of biological fluids in a minimally-invasive, painless, and convenient manner.

It is still another object of the present invention to provide a hollow microneedle array for use in delivery or sensing of drugs or biological fluids or molecules.

### Summary Of The Invention

Microneedle devices for transport of molecules, including drugs and biological molecules, across tissue, and methods for manufacturing the devices, are provided. The microneedle devices permit drug delivery or removal of body fluids at clinically relevant rates across skin or other tissue barriers, with minimal or no damage, pain, or irritation to the tissue. Microneedles can be formed of a variety of materials, including biodegradable or non-biodegradable polymeric materials or metals. In one preferred embodiment, the device includes a means for temporarily securing the microneedle device to the biological barrier to facilitate transport. The device preferably further includes a means for controlling the flow of material through the microneedles. Representative examples of these means include the use of permeable membranes, fracturable impermeable membranes, valves, and pumps, and electrical means.

Methods are provided for making porous, or, preferably, hollow microneedles. A preferred method for making a microneedle includes forming a micromold having sidewalls which define the outer surface of the microneedle. The micromold can be formed, for example, by photolithographically defining one or more holes in a substrate, or by laser based cutting (either serially or by using lithographic projection), or by using a mold-insert. In a preferred embodiment, the method includes electroplating the sidewalls to form the hollow microneedle, and then removing the micromold from the microneedle.

The microneedle device is useful for delivery of fluid material into or across a biological barrier such as skin, wherein the fluid material is delivered from one or more chambers in fluid connection with at least one of the microneedles.

### Brief Description of The Drawings

Figure 1 is a side elevational view of a preferred embodinent of the microneedle device inserted into human skin.
Figures 2a-e are side cross-sectional views of a method for making microneedles.
Figures 3a-g are side cross-sectional views of a method for making a hollow microneedle.
Figures 4a-d are side cross-sectional views illustrating a preferred method for making hollow microneedles.
Figures 5a-d are side cross-sectional views illustrating a preferred method for making hollow silicon microtubes.
Figures 6a-e are side cross-sectional views illustrating a preferred method for making hollow metal microtubes.
Figures 7a-d are side cross-sectional views illustrating a preferred method for making tapered metal microneedles.
Figures 8a-d are side cross-sectional views illustrating a method for making tapered microneedles using laser-formed molds.
Figures 9a-f are side cross-sectional views illustrating a second method for making tapered microneedles using laser-formed molds.

### Detailed Description Of The Invention

### 1. Biological Barriers

The devices disclosed herein are useful in transport of material into or across biological barriers including the skin (or parts thereof); the blood-brain barrier; mucosal tissue (e.g., oral, nasal, ocular, vaginal, methral,gastrointestinal, respiratory); blood vessels; lymphatic vessels; or cell membranes (e.g., for the introduction of material into the interior of a cell or cells). The biological barriers can be in humans or other types of animals, as well as in plants, insects, or other organismes, including bacteria, yeast, fungi, and embryos.

The microneedle devices can be applied to tissue internally with the aid of a catheter or laparoscope. For certain applications, such as for drug delivery to an internal tissue, the devices can be surgically implanted.

The microneedle device disclosed herein is typically applied to skin. The stratum, corneum is the outer layer, generally between 10 and 50 cells, between 10 and 20 µm thick. Unlike other tissue in the body, the stratum corneum contains "cells" (called keratinocytes) filled with bundles of cross-linked keratin and keratohyalin surrounded by an extracelllular matrix of lipids. It is this structure that is believed to give skin its barrier properties, which prevents therapeutic transdermal administration of many drugs. Bellow the stratum corneum is the viable epidermis, which is between 50 and 100 µm thick. The viable epidermis contains no blood vessels, and it exchanges metabolites by diffusion to and from the dermis. Beneath the viable epidermis is the dermis, which is between 1 and 3 mm, thick and contains blood vessels, lymphatics, and nerves.

### 2. The Microneedle Device

The microneedle devices disclosed herein include a substrate; a plurality of microneedles as defined in claim 1 and, optionally, a reservoir for delivery of drugs or collection of analyte, as well as pumps(s), sensor(s), and/or microprocessor(s) to control the interaction of the foregoing.

### a. Substrate

The substrate of the device can be constructed from a variety of materials, including metals, ceramics, semiconductors, organics, polymers, and composites. The substrate includes the base to which the microneedles are attached or integrally formed. A reservoir may also be attached to the substrate.

### b. Microneedle

The microneedles of the device can be constructed from a variety of materials, including metals, ceramics, semiconductors, organics, polymers, and composites. Preferred materials of construction include pharmaceutical grade stainless steel, gold, titanium, nickel, iron, gold, tin, chromium, copper, alloys of these or other metals, silicon, silicon dioxide, and polymers. Representative biodegradable polymers include polymers of hydroxy acids such as lactic acid and glycolic acid polylactide, polyglycolide, polylactide-co-glycolide, and copolymers with PEG, polyanhydrides, poly(ortho)esters, polyurethanes, poly(butyric acid), poly(valeric acid), and poly(lactide-co-caprolactone). Representative non-biodegradable polymers include polycarbonate, polymethacrylic acid, ethylenevinyl acetate, polytetrafluoroethylene (TEFLON^{™}), and polyesters.

Generally, the microneedles should have the mechanical strength to remain intact for delivery of drugs, or serve as a conduit for the collection of biological fluid, while being inserted into the skin, while remaining in place for up to a number of days, and while being removed. In embodiments where the microneedles are formed of biodegradable polymers, however, this mechanical requirement is less stringent, since the microneedles or tips thereof can break off, for example in the skin, and will biodegrade. Nonetheless, even a biodegradable microneedle still needs to remain intact at least long enough for the microneedle to serve its intended purpose (e.g., its conduit function). Therefore, biodegradable microneedles can provide an increased level of safety, as compared to nonbiodegradable ones. The microneedles should be sterilizable using standard methods.

The microneedles can be formed of a porous solid, with or without a sealed coating or exterior portion, or hollow. As used herein, the term "porous" means having pores or voids throughout at least a portion of the microneedle structure, sufficiently large and sufficiently interconnected to permit passage of fluid and/or solid materials through the microneedle. As used herein, the term "hollow" means having one or more substantially annular bores or channels through the interior of the microneedle structure, having a diameter sufficiently large to permit passage of fluid and/or solid materials through the microneedle. The annular bores may extend throughout all or a portion of the needle in the direction of the tip to the base, extending parallel to the direction of the needle or branching or exiting at a side of the needle, as appropriate. A porous microneedle can be hollow. One of skill in the art can select the appropriate porosity and/or bore features required for specific applications. For example, one can adjust the pore size or bore diameter to permit passage of the particular material to be transported through the microneedle device.

The microneedles can have straight or tapered shafts. A hollow microneedle that has a substantially uniform diameter, which needle does not taper to a point, is referred to herein as a "microtube." As used herein, the term "microneedle" includes both microtubes and tapered needles unless otherwise indicated. In a preferred embodiment, the diameter of the microneedle is greatest at the base end of the microneedle and tapers to a point at the end distal the base. The microneedle can also be fabricated to have a shaft that includes both a straight (untapered) portion and a tapered portion.

The microneedles can be formed with shafts that have a circular cross-section in the perpendicular, or the cross-section can be non-circular. For example, the cross-section of the microneedle can be polygonal (e.g. star-shaped, square, triangular), oblong, or another shape. The shaft can have one or more bores. The cross-sectional dimensions typically are between about 10 nm and 1 mm, preferably between 1 micron and 200 microns, and more preferably between 10 and 100 µm. The outer, diameter is typically between about 10 µm and about 100 µm, and the inner diameter is typically between about 3 µm and about 80 µm.

The length of the microneedles typically is between about 1 µm and 1 mm, preferably between 10 microns and 500 microns, and more preferably between 30 and 200 µm. The length is selected for the particular application, accounting for both an inserted and uninserted portion. An array of microneedles can include a mixture of microneedles having, for example, various lengths, outer diameters, inner diameters, cross-sectional shapes, and spacings between the microneedles.

The microneedles can be oriented perpendicular or at an angle to the substrate. Preferably, the microneedles are oriented perpendicular to the substrate so that a larger density of microneedles per unit area of substrate is provided. An array of microneedles can include a mixture of microneedle orientations, heights, or other parameters.

In a preferred embodiment of the device, the substrate and/or microneedles, as well as other components, are formed from flexible materials to allow the device to fit the contours of the biological barrier, such as the skin, vessel walls, or the eye, to which the device is applied. A flexible device facilitates more consistent penetration during use, since penetration can be limited by deviations in the attachment surface. For example, the surface of human skin is not flat due to dermatoglyphics (i.e. tiny wrinkles) and hair.

### c. Reservoir

The microneedle device may include a reservoir in communication with the microneedles. The reservoir can be attached to the substrate by any suitable means. In a preferred embodiment, the reservoir is attached to the back of the substrate (opposite the microneedles) around the periphery, using an adhesive agent (e.g., glue). A gasket may also be used to facilitate formation of a fluid-tight seal.

In a preferred embodiment, the reservoir contains drug, for delivery through the microneedles. The reservoir may be a hollow vessel, a porous matrix, or a solid form including drug which is transported therefrom. The reservoir can be formed from a variety of materials that are compatible with the drug or biological fluid contained therein. Preferred materials include natural and synthetic polymers, metals, ceramics, semiconductors, organics, and composites.

The microneedle device can include one or a plurality of chambers for storing materials to be delivered. In the embodiment having multiple chambers, each can be in fluid connection with all or a portion of the microneedles of the device array. In one embodiment, at least two chambers are used to separately contain drug (e.g., a lyophilized drug, such as a vaccine) and an administration vehicle (e.g., saline) in order to prevent or minimize degradation during storage. Immediately before use, the contents of the chambers are mixed. Mixing can be triggered by any means, including, for example, mechanical disruption (i.e. puncturing or breaking), changing the porosity, or electrochemical degradation of the walls or membranes separating the chambers. In another embodiment, a single device is used to deliver different drugs, which are stored separately in different chambers. In this embodiment, the rate of delivery of each drug can be independently controlled.

In a preferred embodiment, the-reservoir should be in direct contact with the microneedles and have holes through which drug could exit the reservoir and flow into the interior of hollow or porous microneedles. In another preferred embodiment, the reservoir has holes which permit the drug to transport out of the reservoir and onto the skin surface. From there, drug is transported into the skin, either through hollow or porous microneedles, along the sides of solid microneedles, or through pathways created by microneedles in the skin.

### d. Transport Control Components

The microneedle device also must be capable of transporting material across the barrier at a useful rate. For example, the microneedle device must be capable of delivering drug across the skin at a rate sufficient to be therapeutically useful. The device may include a housing with microelectronics and other micromachined structures to control the rate of delivery either according to a preprogrammed schedule or through active interface with the patient, a healthcare professional, or a biosensor. The rate can be controlled by manipulating a variety of factors, including the characteristics of the drug formulation to be delivered (e.g., its viscosity, electric charge, and chemical composition); the dimensions of each microneedle (e.g., its outer diameter and the area of porous or hollow openings); the number of microneedles in the device; the application of a driving force (e.g., a concentration gradient, a voltage gradient, a pressure gradient); and the use of a valve.

The rate also can be controlled by interposing between the drug in the reservoir and the opening(s) at the base end of the microneedle polymeric or other materials selected for their diffusion characteristics. For example, the material composition and layer thickness can be manipulated using methods known in the art to vary the rate of diffusion of the drug of interest through the material, thereby controlling the rate at which the drug flows from the reservoir through the microneedle and into the tissue.

Transportation of molecules through the microneedles can be controlled or monitored using, for example, various combinations of valves, pumps, sensors, actuators, and microprocessors. These components can be produced using standard manufacturing or microfabrication techniques. Actuators that may be useful with the microneedle devices disclosed herein include micropumps, microvalves, and positioners. In a preferred embodiment, a microprocessor is programmed to control a pump or valve, thereby controlling the rate of delivery.

Flow of molecules through the microneedles can occur based on diffusion, capillary action, or can be induced using conventional mechanical pumps or nonmechanical driving forces, such as electroosmosis or electrophoresis, or convection. For example, in electroosmosis, electrodes are positioned on the biological barrier surface, one or more microneedles, and/or the substrate adjacent the needles, to create a convective flow which carries oppositely charged ionic species and/or neutral molecules toward or into the biological barrier. In a preferred embodiment, the microneedle device is used in combination with another mechanism that enhances the permeability of the biological barrier, for example by increasing cell uptake or membrane disruption, using electric fields, ultrasound, chemical enhancers, vacuum viruses, pH, heat and/or light.

Passage of the microneedles, or drug to be transported via the microneedles, can be manipulated by shaping the microneedle surface, or by selection of the material forming the microneedle surface (which could be a coating rather than the microneedle per se). For example, one or more grooves on the outside surface of the microneedles can be used to direct the passage of drug, particularly in a liquid state. Alternatively, the physical surface properties of the microneedle can be manipulated to either promote or inhibit transport of material along the microneedle surface, such as by controlling hydrophilicity or hydrophobicity.

The flow of molecules can be regulated using a wide range of valves or gates. These valves can be the type that are selectively and repeatedly opened and closed, or they can be single-use types. For example, in a disposable, single-use drug delivery device, a fracturable barrier or one-way gate may be installed in the device between the reservoir and the opening of the microneedles. When ready to use, the barrier can be broken or gate opened to permit flow through the microneedles. Other valves or gates used in the microneedle devices can be activated thermally, electrochemically, mechanically, or magnetically to selectively initiate, modulate, or stop the flow of molecules through the needles. In a preferred embodiment, flow is controlled by using a rate-limiting membrane as a "valve."

The microneedle devices can further include a flowmeter or other means to monitor flow through the microneedles and to coordinate use of the pumps and valves.

### e. Sensors

Useful sensors may include sensors of pressure, temperature, chemicals, and/or electro-magnetic fields. Biosensors can be located on the microneedle surface, inside a hollow or porous microneedle, or inside a device in communication with the body tissue via the microneedle (solid, hollow, or porous). These microneedle biosensors can include four classes of principal transducers: potentiometric, amperometric, optical, and physiochemical. An amperometric sensor monitors currents generated when electrons are exchanged between a biological system and an electrode. Blood glucose sensors frequently are of this type.

The microneedle may function as a conduit for fluids, solutes, electric charge, light, or other materials. In one embodiment, hollow microneedles can be filled with a substance, such as a gel, that has a sensing functionality associated with it. In an application for sensing based on binding to a substrate or reaction mediated by an enzyme, the substrate or enzyme can be immobilized in the needle interior, which would be especially useful in a porous needle to create an integral needle/sensor.

Wave guides can be incorporated into the microneedle device to direct light to a specific location, or for detection, for example, using means such as a pH dye for color evaluation. Similarly, heat, electricity, light or other energy forms may be precisely transmitted to directly stimulate, damage, or heal a specific tissue or intermediary (e.g., tattoo remove for dark skinned persons), or diagnostic purposes, such as measurement of blood glucose based on IR spectra or by chromatographic means, measuring a color change in the presence of immobilized glucose oxidase in combination with an appropriate substrate.

### f. Attachment Features

A collar or flange also can be provided with the device, for example, around the periphery of the substrate or the base. It preferably is attached to the device, but alternatively can be formed as an integral part of the substrate, for example by forming microneedles only near the center of an "oversized" substrate. The collar can also emanate from other parts of the device. The collar can provide an interface to attach the microneedle array to the rest of the device, and can facilitate handling of the smaller devices.

In a preferred embodiment, the microneedle device includes an adhesive to temporarily secure the device to the surface of the biological barrier. The adhesive can be essentially anywhere on the device to facilitate contact with the biological barrier. For example, the adhesive can be on the surface of the collar (same side as microneedles), on the surface of the substrate between the microneedles (near the base of the microneedles), or a combination thereof.

### g. Transdermal Microneedle Device

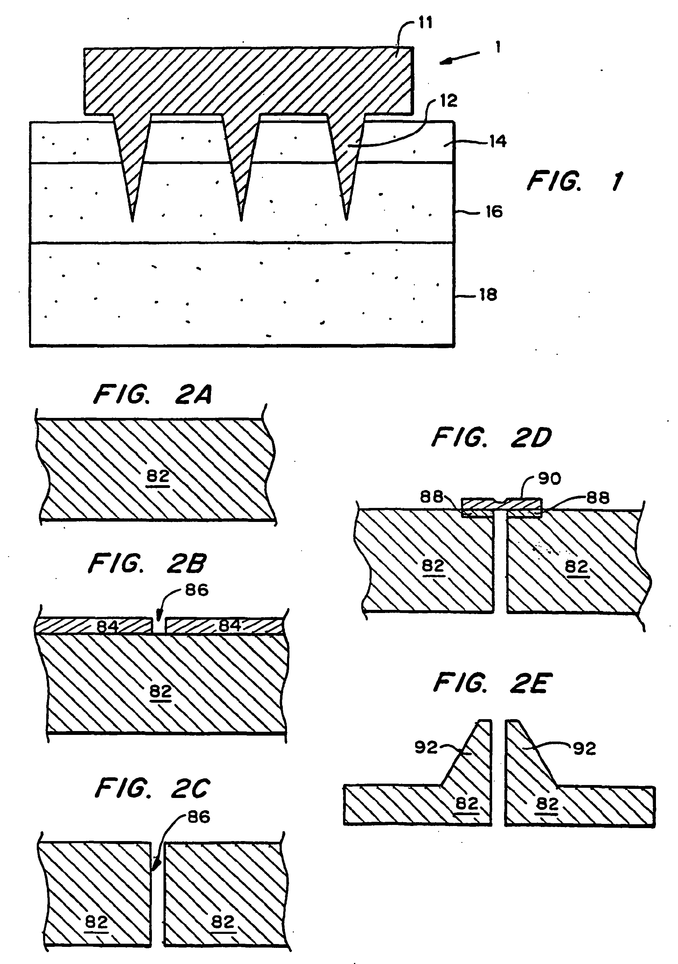

Figure 1 is a side elevational view of a schematic of a preferred embodiment of the microneedle device inserted into skin. The device **10** includes an upper portion or substrate **11** from which a plurality of microneedles **12** protrude. The height of the upper portion **11** is between about 1 µm and 1 cm, and the width of the upper portion is between about 1 mm and 10 cm. The upper portion **11** of the device can be solid or hollow, and may include multiple compartments. In a preferred embodiment for drug delivery, the upper portion **11** contains one or more drugs to be delivered. It is also preferred that the upper portion include one or more sensors and/or an apparatus (e.g., pump or electrode) to drive (provide/direct the force) transport of the drug or other molecules.

The height (or length) of the microneedles **12** generally is between about 10 µm and 1 mm. The diameter and length both affect pain as well as functional properties of the needles. In transdermal applications, the "insertion depth" of the microneedles 12 is preferably less than about 100 µm, more preferably about 30 µm, so that insertion of the microneedles **12** into the skin through the stratum corneun 14 does not penetrate past the epidermis **16** into the dermis **18** (as described below), thereby avoiding contacting nerves and reducing the potential for causing pain. In such applications, the actual length of the microneedles may be longer, since the portion of the microneedles distal the tip may not be inserted into the skin; the uninserted length depends on the particular device design and configuration. The actual (overall) height or length of microneedles **12** should be equal to the insertion depth plus the uninserted length.

The diameter of each microneedle **12** generally is between about 10 nm and 1 mm, and preferably leaves a residual hole (following microneedle insertion and withdrawal) of less than about 1 µm, to avoid making a hole which would allow bacteria to enter the penetration wound. The actual microneedle diameter should be larger than 1 µm, since the hole likely will contract following withdrawal of the microneedle. The diameter of microneedle **12** more preferably is between about 1 µm and 100 µm. The microneedles **12** can be porous, and can include one or more bores connected to upper portion **11**.

### 3. Methods of Making Microneedle Devices

The microneedle devices are made by microfabrication processes, by creating small mechanical structures in silicon, metal, polymers, and other materials. These microfabrication processes are based on well-established methods used to make integrated circuits, electronic packages and other microelectronic devices, augmented by additional methods used in the field of micromachining. The microneedle devices can have dimensions as small as a few nanometers and can be mass-produced at low per-unit costs.

### a. Microfabrication Processes

Microfabrication processes that may be used in making the microneedles disclosed herein include lithography; etching techniques, such as wet chemical, dry, and photoresist removal; thermal oxidation of silicon; electroplating and electroless plating; diffusion processes, such as boron, phosphorus, arsenic, and antimony diffusion; ion implantation; film deposition, such as evaporation (filament, electron beam, flash, and shadowing and step coverage), sputtering, chemical vapor deposition (CVD), epitaxy (vapor phase, liquid phase, and molecular beam), electroplating, screen printing, lamination, stereolithography, laser machining, and laser ablation (including projection ablation). *See generally* Jaeger, Introduction to Microelectronic Fabrication (Addison-Wesley Publishing Co., Reading MA 1988); Runyan, et al., Semiconductor Integrated Circuit Processing Technology (Addison-Wesley Publishing Co., Reading MA 1990); Proceedings of the IEEE Micro Electro Mechanical Systems Conference 1987-1998; Rai-Choudhury, ed., Handbook of Microlithography, Micromachining & Microfabrication (SPIE Optical Engineering Press, Bellingham, WA 1997).

The following methods are preferred for making microneedles.

### i. electrochemical etching of silicon

In this method, electrochemical etching of solid silicon to porous silicon is used to create extremely fine (on the order of 0.01 µm) silicon networks which can be used as piercing structures. This method uses electrolytic anodization of silicon in aqueous hydrofluoric acid, potentially in combination with light, to etch channels into the silicon. By varying the doping concentration of the silicon wafer to be etched, the electrolytic potential during etching, the incident light intensity, and the electrolyte concentration, control over the ultimate pore structure can be achieved. The material not etched (i.e. the silicon remaining) forms the microneedles. This method has been used to produce irregular needle-type structures measuring tens of nanometers in width.

### ii. plasma etching

This process uses deep plasma etching of silicon to create microneedles with diameters on the order of 0.1 µm or larger. Needles are patterned directly using photolithography, rather than indirectly by controlling the voltage (as in electrochemical etching), thus providing greater control over the final microneedle geometry.

In this process, an appropriate masking material (e.g., metal) is deposited onto a silicon wafer substrate and patterned into dots having the diameter of the desired microneedles. The wafer is then subjected to a carefully controlled plasma based on fluorine/oxygen chemistries to etch very deep, high aspect ratio trenches into the silicon. *See, e.g.,* Jansen, et al., "The Black Silicon Method IV: The Fabrication of Three-Dimensional Structures in Silicon with High Aspect Ratios for Scanning Probe Microscopy and Other Applications," IEEE Proceedings of Micro Electro Mechanical Systems Conference, pp. 88-93 (1995). Those regions protected by the metal mask remain and form the needles. This method is further described in Example 1 below.

### iii. electroplating

In this process, a metal layer is first evaporated onto a planar substrate. A layer of photoresist is then deposited onto the metal to form a patterned mold which leaves an exposed-metal region in the shape of needles. By electroplating onto the exposed regions of the metal seed layer, the mold bounded by photoresist can be filled with electroplated material. Finally, the substrate and photoresist mold are removed, leaving the finished microneedle array. The microneedles produced by this process generally have diameters on the order of 1 µm or larger. *See, e.g.,* Frazier, et al., "Two dimensional metallic microelectrode arrays for extracellular stimulation and recording of neurons", IEEE Proceedings of the Micro Electro Mechanical Systems Conference, pp. 195-200 (1993).

### iv other processes

Another method for forming microneedles made of silicon or other materials is to use microfabrication techniques such as photolithography, plasma etching, or laser ablation to make a mold form (A), transferring that mold form to other materials using standard mold transfer techniques, such as embossing or injection molding (B), and reproducing the shape of the original mold form (A) using the newly-created mold (B) to yield the final microneedles (C). Alternatively, the creation of the mold form (A) could be skipped and the mold (B) could be microfabricated directly, which could then be used to create the final microneedles (C).

Another method of forming solid silicon microneedles is by using epitaxial growth on silicon substrates, as is utilized by Containerless Research, Inc. (Evanston, Illinois, USA) for its products.

### b. Hollow or Porous Microneedles

In a preferred embodiment, microneedles are made with pores or other pathways through which material may be transported. The following descriptions outline representative methods for fabricating either porous or hollow microneedles.

### i. porous microneedles

Rather than having a single, well-defined hole down the length of the needle, porous needles are filled with a network of channels or pores which allow conduction of fluid or energy through the needle shaft. It has been shown that by appropriate electrochemical oxidation of silicon, pore arrays with high aspect ratios and a range of different pore size regimes can be formed; these pore regimes are defined as (1) microporous regime with average pore dimensions less than 2 nm, (2) mesoporous regime with average pore sizes of between 2 nm and 50 nm, and (3) macroporous regime with pores greater than 50 nm. The mesoporous and macroporous regimes are expected to be most useful for drug delivery. Two approaches to porous needles are generally available, either (a) the silicon wafer is first made porous and then etched as described above to form needles or (b) solid microneedles are etched and then rendered porous, for example, by means of electrochemical oxidation, such as by anodization of a silicon substrate in a hydrofluoric acid electrolyte. The size distribution of the etched porous structure is highly dependent on several variables, including doping kind and illumination conditions, as detailed in Lehmann, "Porous Silicon-A New Material for MEMS", IEEE Proceedings of the Micro Electro Mechanical Systems Conference, pp. 1-6 (1996). Porous polymer or metallic microneedles can be formed, for example, by micromolding a polymer containing a volatilizable or leachable material, such as a volatile salt, dispersed in the polymer or metal, and then volatilizing or leaching the dispersed material, leaving a porous polymer matrix in the shape of the microneedle.

### ii. hollow needles

Three-dimensional arrays of hollow microneedles can be fabricated, for example, using combinations of dry etching processes (Laermer, et al., "Bosch Deep Silicon Etching: Improving Uniformity and Etch Rate for Advanced MEMS Applications," Micro Electro Mechanical Systems, Orlando, F1, USA, (Jan. 17-21, 1999); Despont et al., "High-Aspect-Ratio, Ultrathick, Negative-Tone Near-UV Photoresist for MEMS", Proc. of IEEE 10th Annual International Workshop on MEMS, Nagoya, Japan, pp. 518-522 (Jan. 26-30, 1997)); micromold creation in lithographically-defined and/or laser ablated polymers and selective sidewall electroplating; or direct micromolding techniques using epoxy mold transfers.

One or more distinct and continuous pathways are created through the interior of microneedles. In a preferred embodiment, the microneedle has a single annular pathway along the center axis of the microneedle. This pathway can be achieved by initially chemically or physically etching the holes in the material and then etching away microneedles around the hole. Alternatively, the microneedles and their holes can be made simultaneously or holes can be etched into existing microneedles. As another option, a microneedle form or mold can be made, then coated, and then etched away, leaving only the outer coating to form a hollow microneedle. Coatings can be formed either by deposition of a film or by oxidation of the silicon microneedles to a specific thickness, followed by removal of the interior silicon. Also, holes from the backside of the wafer to the underside of the hollow needles can be created using a front-to-backside infrared alignment followed by etching from the backside of the wafer.

### a. silicon microneedles

One method for hollow needle fabrication is to replace the solid mask used in the formation of solid needles by a mask that includes a solid shape with one or more interior regions of the solid shape removed. One example is a "donut-shaped" mask. Using this type of mask, interior regions of the needle are etched simultaneously with their side walls. Due to lateral etching of the inner side walls of the needle, this may not produce sufficiently sharp walls. In that case, two plasma etches may be used, one to form the outer walls of the microneedle (i.e., the 'standard' etch), and one to form the inner hollow core (which is an extremely anisotropic etch, such as in inductively-coupled-plasma "ICP" etch). For example, the ICP etch can be used to form the interior region of the needle followed by a second photolithography step and a standard etch to form the outer walls of the microneedle. Figure 2a represents a silicon wafer **82** with a patterned photoresist layer **84** on top of the wafer **82.** The wafer **82** is anisotropically etched (Figure 2b) to form a cavity **86** through its entire thickness (Figure 2c). The wafer **82** is then coated with a chromium layer **88** followed by a second photoresist layer **90** patterned so as to cover the cavity **86** and form a circular mask for subsequent etching (Figure 2d). The wafer **82** is then etched by a standard etch to form the outer tapered walls **92** of the microneedle (Figure 2e).

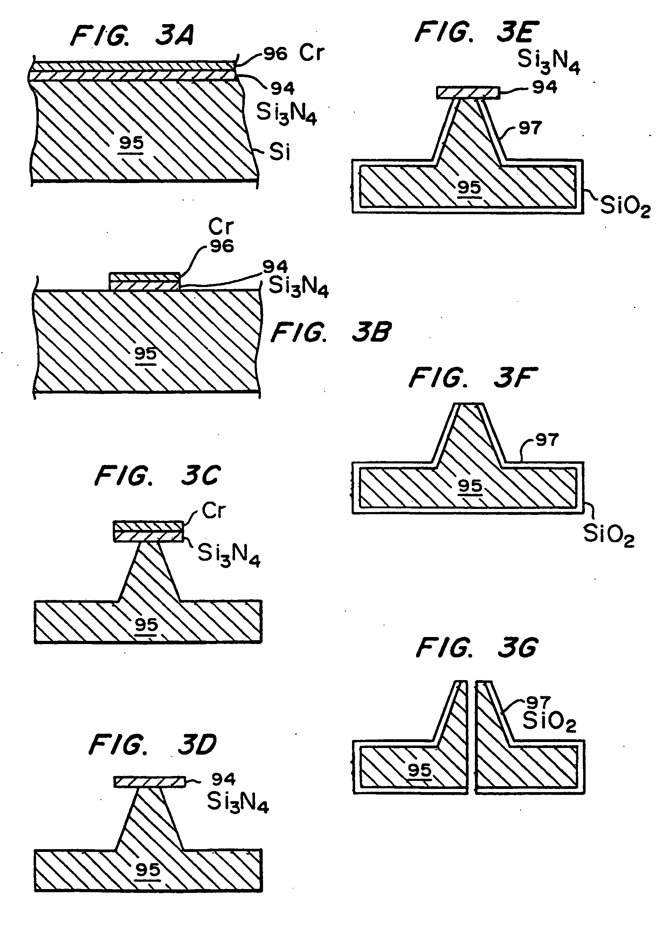

Alternatively, this structure can be achieved by substituting the chromium mask used for the solid microneedles described in Example 1 by a silicon nitride layer **94** on the silicon substrate **95** covered with chromium **96,** deposited as shown in Figure 3a and patterned as shown in Figure 3b. Solid microneedles are then etched as described in Example 1 as shown Figure 3c, the chromium **96** is stripped (Figure 3d), and the silicon **95** is oxidized to form a thin layer of silicon dioxide **97** on all exposed silicon surfaces (Figure 3e). The silicon nitride layer **94** prevents oxidation at the needle tip. The silicon nitride **94** is then stripped (Figure 3f), leaving exposed silicon at the tip of the needle and oxide-covered silicon **97** everywhere else. The needle is then exposed to an ICP plasma which selectively etches the inner sidewalls of the silicon **95** in a highly anisotropic manner to form the interior hole of the needle (Figure 3g).

Another method uses the solid silicon needles described previously as 'forms' around which the actual needle structures are deposited. After deposition, the forms are etched away, yielding the hollow structures. Silica needles or metal needles can be formed using different methods. Silica needles can be formed by creating needle structures similar to the ICP needles described above prior to the oxidation described above. The wafers are then oxidized to a controlled thickness, forming a layer on the shaft of the needle form which will eventually become the hollow microneedle. The silicon nitride is then stripped and the silicon core selectively etched away (e.g., in a wet alkaline solution) to form a hollow silica microneedle.

In a preferred embodiment, an array of hollow silicon microtubes is made using deep reactive ion etching combined with a modified black silicon process in a conventional reactive ion etcher, as described in Example 3 below. First, arrays of circular holes are patterned through photoresist into SiO₂, such as on a silicon wafer. Then the silicon can be etched using deep reactive ion etching (DRIE) in an inductively coupled plasma (ICP) reactor to etch deep vertical holes. The photoresist was then removed. Next, a second photolithography step patterns the remaining SiO₂ layer into circles concentric to the holes, leaving ring shaped oxide masks surrounding the holes. The photoresist is then removed and the silicon wafer again deep silicon etched, such that the holes are etched completely through the wafer (inside the SiO₂ ring) and simultaneously the silicon is etched around the SiO₂ ring leaving a cylinder.

This latter process can be varied to produce hollow, tapered microneedles. After an array ofholes is fabricated as described above, the photoresist and SiO₂ layers are replaced with conformal DC sputtered chromium rings. The second ICP etch is replaced with a SF₆/O₂ plasma etch in a reactive ion etcher (RIE), which results in positively sloping outer sidewalls. Henry, et al., "Micromachined Needles for the Transdermal Delivery of Drugs," Micro Electro Mechanical Systems, Heidelberg, Germany, pp. 494-498 (Jan. 26-29, 1998).

### b. metal microneedles

Metal needles can be formed by physical vapor deposition of appropriate metal layers on solid needle forms, which can be made of silicon using the techniques described above, or which can be formed using other standard mold techniques such as embossing or injection molding. The metals are selectively removed from the tips of the needles using electropolishing techniques, in which an applied anodic potential in an electrolytic solution will cause dissolution of metals more rapidly at sharp points, due to concentration of electric field lines at the sharp points. Once the underlying silicon needle forms have been exposed at the tips, the silicon is selectively etched away to form hollow metallic needle structures. This process could also be used to make hollow needles made from other materials by depositing a material other than metal on the needle forms and following the procedure described above.

A preferred method of fabricating hollow metal microneedles utilizes micromold plating techniques, which are described as follows and in Examples 4 and 5. In a method for making metal microtubes, which does not require dry silicon etching, a photo-defined mold first is first produced, for example, by spin casting a thick layer, typically 150 µm, of an epoxy (e.g., SU-8) onto a substrate that has been coated with a thin sacrificial layer, typically about 10 to 50 nm. Arrays of cylindrical holes are then photolithographically defined through the epoxy layer, which typically is about 150 µm thick. (Despont, et al., "High-Aspect-Ratio, Ultrathick, Negative-Tone Near-UV Photoresist for MEMS," Proc. of IEEE 10th Annual International Workshop on MEMS, Nagoya, Japan, pp. 518-522 (Jan. 26-30, 1997)). The diameter of these cylindrical holes defines the outer diameter of the tubes. The upper surface of the substrate, the sacrificial layer, is then partially removed at the bottom of the cylindrical holes in the photoresist. The exact method chosen depends on the choice of substrate. For example, the process has been successfully performed on silicon and glass substrates (in which the upper surface is etched using isotropic wet or dry etching techniques) and copper-clad printed wiring board substrates. In the latter case, the copper laminate is selectively removed using wet etching. Then a seed layer, such as Ti/Cu/Ti (e.g., 30 nm/200 nm/30 nm), is conformally DC sputter-deposited onto the upper surface of the epoxy mold and onto the sidewalls of the cylindrical holes. The seed layer should be electrically isolated from the substrate. Subsequently, one or more electroplatable metals or alloys, such as Ni, NiFe, Au, Cu, or Ti, are electroplated onto the seed layer. The surrounding epoxy is then removed, leaving microtubes which-each have an interior annular hole that extends through the base metal supporting the tubes. The rate and duration of electroplating is controlled in order to define the wall thickness and inner diameter of the microtubes. In one embodiment, this method was used to produce microtubes having a height of between about 150 and 250 µm, an outer diameter of between about 40 and 120 µm, and an inner diameter of between about 30 and 110 µm (i.e., having a wall thickness of 10 µm). In a typical array, the microtubes have a tube center-to-center spacing of about 150 µm, but can vary depending on the desired needle density.

A variation of this method is preferred for forming tapered microneedles. As described above, photolithography yields holes in the epoxy which have vertical sidewalls, such that the resulting shafts of the microneedles are straight, not tapered. This vertical sidewall limitation can be overcome by molding a preexisting 3D structure, i.e., a mold-insert. The subsequent removal of the mold-insert leaves a mold which can be surface plated similarly to the holes produced by photolithography described above.

Alternatively, non-vertical sidewalls can be produced directly in the polymeric mold into which electroplating will take place. For example, conventional photoresists known in the art can be exposed and developed in such as way as to have the surface immediately adjacent to the mask be wider than the other surface. Specialized greyscale photoresists in combination with greyscale masks can accomplish the same effect. Laser-ablated molds can also be made with tapered sidewalls, e.g., by optical adjustment of the beam (in the case of serial hole fabrication) or of the reticle or mold during ablation (in the case of projection ablation).

To form hollow tapered microneedles, the mold-insert is an array of solid silicon microneedles, formed as described in Henry, et al., "Micromachined Needles for the Transdermal Delivery of Drugs," Micro Electro Mechanical Systems, Heidelberg, Germany, Jan. 26-29, pp. 494-498 (1998). First, a layer of a material, such as an epoxy (e.g., SU-8 or a polydimethylsiloxane ("PDMS")), is spin cast onto the array of silicon microneedles to completely blanket the entire array. The epoxy settles during pre-bake to create a planar surface above the silicon needle tips; the material is then fully pre-baked, photolithographically cross-linked, and post-baked.

The upper surface of the epoxy is then etched away, for example with an O₂/CHF₃ plasma, until the needle tips are exposed, preferably leaving between about 1 and 5 µm of tip protruding from the epoxy. The silicon is then selectively removed, for example by using a SF₆ plasma or a HNO₃/HF solution. The remaining epoxy micromold is the negative of the microneedles and has a small diameter hole where the tip of the microneedle formerly protruded.

After the removal of the silicon, a seed layer, such as Ti-Cu-Ti, is conformally sputter-deposited onto the epoxy micromold. Following the same process sequence described for hollow metal microtubes, one or more electroplatable metals or alloys, such as Ni, NiFe, Au, or Cu, are electroplated onto the seed layer. Finally, the epoxy is removed, for example by using an O₂/CHF₃ plasma, leaving an array of hollow metal microneedles. An advantage of using PDMS in this application is that the micromold can be physically removed from the silicon mold insert by mechanical means, such as peeling, without damaging the silicon mold insert, thus allowing the silicon mold insert to be reused. Furthermore, the electroplated microneedles can be removed from the PDMS mold by mechanical means, for example by peeling, thereby allowing the PDMS to also be reused. In a preferred embodiment, this method is used to produce microneedles having a height of between about 150 and 250 µm, an outer diameter of between about 40 and 120 µm, and an inner diameter of between about 50 and 100 µm. In a typical array, the microtubes have a tube center-to-center spacing of about 150 µm, but can vary depending on the desired needle density. The microneedles are 150 µm in height with a base diameter of 80 µm, a tip diameter of 10 µm, and a needle-to-needle spacing of 150 µm_{.}

### c. silicon dioxide microneedles

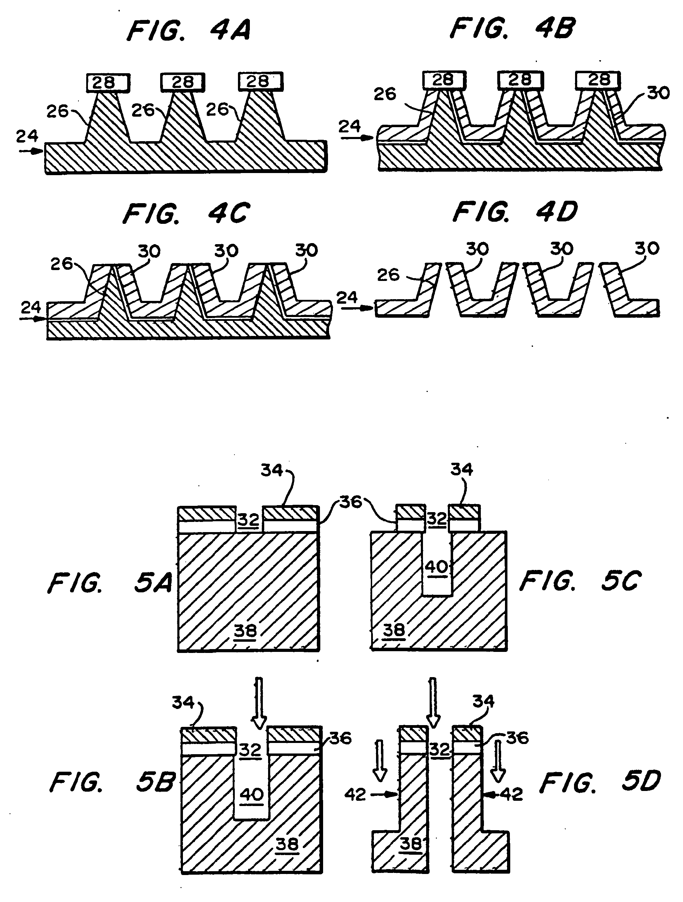

Hollow microneedles formed of silicon dioxide can be made by oxidizing the surface of the silicon microneedle forms (as described above), rather than depositing a metal and then etching away the solid needle forms to leave the hollow silicon dioxide structures. This method is illustrated in **Figures 4a-4d. Figure 4a** shows an array **24** of needle forms **26** with masks **28** on their tips. In Figure 4b, the needle forms **26** have been coated with a layer **30** of metal, silicon dioxide or other material. Figure 4c shows the **coated needle forms 26 with** the masks **28** removed. Finally, in Figure 4d, the needle forms **26** have been etched away, leaving hollow needles 30 made of metal, silicon dioxide, or other materials.

In one embodiment, hollow or porous microneedles are provided with longitudinal grooves or other modifications to the exterior surface of the microneedles. Grooves, for example, should be useful in directing the flow of molecules along the outside of microneedles.

### d. polymer microneedles

In a preferred method, polymeric microneedles are made using microfabricated molds. For example, the epoxy molds can be made as described above and injection molding techniques can be applied to form the microneedles in the molds (Weber, et al., "Micromolding - a powerful tool for the large scale production of precise microstructures", Proc. SPIE - International Soc. Optical Engineer. 2879,156-167 (1996); Schift, et al., "Fabrication of replicated high precision insert elements for micro optical bench arrangements" Proc. SPIB - International Soc. Optical Engineer. 3513, 122-134 (1998). These micromolding techniques are preferred over other techniques described herein, since they can provide relatively less expensive replication, i.e. lower cost of mass production. In a preferred embodiment, the polymer is biodegradable.

### 4. Microneedle Device Applications

The device may be used for single or multiple uses for rapid transport across a biological barrier or may be left in place for longer times (e.g., hours or days) for long-term transport of molecules. Depending on the dimensions of the device, the application site, and the route in which the device is introduced into (or onto) the biological barrier, the device may be used to introduce or remove molecules at specific locations.

As discussed above, Figure 1 shows a side elevational view of a schematic of a preferred embodiment of the microneedle device **10** in a transdermal application. The device **10** is applied to the skin such that the microneedles **12** penetrate through the stratum corneum and enter the viable epidermis so that the tip of the microneedle at least penetrates into the viable epidermis, In a preferred embodiment, drug molecules in a reservoir within the upper portion **11** flow through or around the microneedles and into the viable epidermis, where the drug molecules then diffuse into the dermis for local treatment or for transport through the body.

To control the transport of material out of or into the device through the microneedles, a variety of forces or mechanisms can be employed. These include pressure gradients, concentration gradients, electricity, ultrasound, receptor binding, heat, chemicals, and chemical reactions. Mechanical or other gates in conjunction with the forces and mechanisms described above can be used to selectively control transport of the material.

In particular embodiments, the device should be "user-friendly." For example, in some transdermal applications, affixing the device to the skin should be relatively simple, and not require special skills. This embodiment of a microneedle may include an array of microneedles attached to a housing containing drug in an internal reservoir, wherein the housing has a bioadhesive coating around the microneedles. The patient can remove a peel-away backing to expose an adhesive coating, and then press the device onto a clean part of the skin, leaving it to administer drug over the course of, for example, several days.

### a. Drug Delivery

Essentially any drug or other bioactive agents can be delivered using these devices. Drugs can be proteins, enzymes, polysaccharides, polynucleotide molecules, and synthetic organic and inorganic compounds. Representative agents include antiinfectives, hormones, such as insulin, growth regulators, drugs regulating cardiac action or blood flow, and drugs for pain control. The drug can be for local treatment or for regional or systemic therapy. The following are representative examples, and disorders they are used to treat:

Calcitonin, osteoporosis; Enoxaprin, anticoagulant; Etanercept, rheumatoid arthritis; Erythropoietin, anemia; Fentanyl, postoperative and chronic pain; Filgrastin, low white blood cells from chemotherapy; Heparin, anticoagulant; Insulin, human, diabetes; Interferon Beta 1a, multiple sclerosis; Lidocaine, local anesthesia; Somatropin, growth hormone; and Sumatriptan, migraine headaches.

In this way, many drugs can be delivered at a variety of therapeutic rates. The rate can be controlled by varying a number of design factors, including the outer diameter of the microneedle, the number and size of pores or channels in each microneedle, the number of microneedles in an array, the magnitude and frequency of application of the force driving the drug through the microneedle and/or the holes created by the microneedles. For example, devices designed to deliver drug at different rates might have more microneedles for more rapid delivery and fewer microneedles for less rapid delivery. As another example, a device designed to deliver drug at a variable rate could vary the driving force (e.g., pressure gradient controlled by a pump) for transport according to a schedule which was pre-programmed or controlled by, for example, the user or his doctor. The devices can be affixed to the skin or other tissue to deliver drugs continuously or intermittently, for durations ranging from a few seconds to several hours or days.

One of skill in the art can measure the rate of drug delivery for particular microneedle devices using *in vitro* and *in vivo* methods known in the art. For example, to measure the rate of transdermal drug delivery, human cadaver skin mounted on standard diffusion chambers can be used to predict actual rates. *See* Hadgraft & Guy, eds., Transdermal Drug Delivery: Developmental Issues and Research Initiatives (Marcel Dekker, New York 1989); Bronaugh & Maibach, Percutaneous Absorption, Mechanisms--Methodology--Drug Delivery (Marcel Dekker, New York 1989). After filling the compartment on the dermis side of the diffusion chamber with saline, a microneedle array is inserted into the stratum corneum; a drug solution is placed in the reservoir of the microneedle device; and samples of the saline solution are taken over time and assayed to determine the rates of drug transport.

In an alternate embodiment, biodegradable or non-biodegradable microneedles can be used as the entire drug delivery device, where biodegradable microneedles are a preferred embodiment. For example, the microneedles may be formed of a biodegradable polymer containing a dispersion of an active agent for local or systemic delivery. The agent could be released over time, according to a profile determined by the composition and geometry of the microneedles, the concentration of the drug and other factors. In this way, the drug reservoir is within the matrix of one or more of the microneedles.

In another alternate embodiment, these microneedles may be purposefully sheared off from the substrate after penetrating the biological barrier. In this way, a portion of the microneedles would remain within or on the other side of the biological barrier and a portion of the microneedles and their substrate would be removed from the biological barrier. In the case of skin, this could involve inserting an array into the skin, manually or otherwise breaking off the microneedles tips and then remove the base of the microneedles. The portion of the microneedles which remains in the skin or in or across another biological barrier could then release drug over time according to a profile determined by the composition and geometry of the microneedles, the concentration of the drug and other factors. In a preferred embodiment, the microneedles are made of a biodegradable polymer. The release of drug from the biodegradable microneedle tips can be controlled by the rate of polymer degradation. Microneedle tips can release drugs for local or systemic effect, or other agents, such as perfume, insect repellent and sun block.

Microneedle shape and content can be designed to control the breakage of microneedles. For example, a notch can be introduced into microneedles either at the time of fabrication or as a subsequent step. In this way, microneedles would preferentially break at the site of the notch. Moreover, the size and shape of the portion of microneedles which break off can be controlled not only for specific drug release patterns, but also for specific interactions with cells in the body. For example, objects of a few microns in size are known to be taken up by macrophages. The portions of microneedles that break off can be controlled to be bigger or smaller than that to prevent uptake by macrophages or can be that size to promote uptake by macrophages, which can be desirable for delivery of vaccines.

### b. Diagnostic Sensing of Body Fluids (Biosensors)

One embodiment of the devices described herein may be used to remove material from the body across a biological barrier, i.e. for minimally invasive diagnostic sensing. For example, fluids can be transported from interstitial fluid in a tissue into a reservoir in the upper portion of the device. The fluid can then be assayed while in the reservoir or the fluid can be removed from the reservoir to be assayed, for diagnostic or other purposes. For example, interstitial fluids can be removed from the epidermis across the stratum corneum to assay for glucose concentration, which should be useful in aiding diabetics in determining their required insulin dose. Other substances or properties that would be desirable to detect include lactate (important for athletes), oxygen, pH, alcohol, tobacco metabolites, and illegal drugs (important for both medical diagnosis and law enforcement).

The sensing device can be in or attached to one or more microneedles, or in a housing adapted to the substrate: Sensing information or signals can be transferred optically (e.g., refractive index) or electrically (e.g., measuring changes in electrical impedance, resistance, current, voltage, or combination thereof). For example, it may be useful to measure a change as a function of change in resistance of tissue to an electrical current or voltage, or a change in response to channel binding or other criteria (such as an optical change) wherein different resistances are calibrated to signal that more or less flow of drug is needed, or that delivery has been completed.

In one embodiment, one or more microneedle devices can be used for (1) withdrawal of interstitial fluid, (2) assay of the fluid, and/or (3) delivery of the appropriate amount of a therapeutic agent based on the results of the assay, either automatically or with human intervention. For example, a sensor delivery system may be combined to form, for example, a system which withdraws bodily fluid, measures its glucose content, and delivers an appropriate amount of insulin. The sensing or delivery step also can be performed using conventional techniques, which would be integrated into use of the microneedle device. For example, the microneedle device could be used to withdraw and assay glucose, and a conventional syringe and needle used to administer the insulin, or *vice versa.*

In an alternate embodiment, microneedles may be purposefully sheared off from the substrate after penetrating the biological barrier, as described above. The portion of the microneedles which remain within or on the other side of the biological barrier could contain one or more biosensors. For example, the sensor could change color as its output. For microneedles sheared off in the skin, this color change could be observed through the skin by visual inspection or with the aid of an optical apparatus.

Other than transport of drugs and biological molecules, the microneedles may be used to transmit or transfer other materials and energy forms, such as light, electricity, heat, or pressure. The microneedles, for example, could be used to direct light to specific locations within the body, in order that the light can directly act on a tissue or on an intermediary, such as light-sensitive molecules in photodynamic therapy. The micrconeedles can also be used for aerosolization or delivery for example directly to a. mucosal surface in the nasal or buccal regions or to the pulmonary system.

The microneedle devices disclosed herein also should be useful for controlling transport across tissues other than skin. For example, microneedles can be inserted into the eye across, for example, conjunctiva, sclera, and/or cornea, to facilitate delivery of drugs into the eye. Similarly, microneedles inserted into the eye can facilitate transport of fluid out of the eye, which may be of benefit for treatment of glaucoma. Microneedles may also be inserted into the buccal (oral), nasal, vaginal, or other accessible mucosa to facilitate transport into, out of, or across those tissues. For example, a drug may be delivered across the buccal mucosa for local treatment in the mouth or for systemic uptake and delivery. As another example, microneedle devices may be used internally within the body on, for example, the lining of the gastrointestinal tract to facilitate uptake of orally-ingested drugs or the lining of blood vessels to facilitate penetration of drugs into the vessel wall. For example, cardiovascular applications include using microneedle devices to facilitate vessel distension or immobilization, similarly to a stent, wherein the microneedles/substrate can function as a "staple-like" device to penetrate into different tissue segments and hold their relative positions for a period of time to permit tissue regeneration. This application could be particular useful with biodegradable devices. These uses may involve invasive procedures to introduce the microneedle devices into the body or could involve swallowing, inhaling, injecting or otherwise introducing the devices in a non-invasive or minimally-invasive manner.

The present invention will be further understood with reference to the following non-limiting examples.

### Comparative Example 1: Fabrication of Solid Silicon Microneedles

A chromium masking material was deposited onto silicon wafers and patterned into dots having a diameter approximately equal to the base of the desired microneedles. The wafers were then loaded into a reactive ion etcher and subjected to a carefully controlled plasma based on fluorine/oxygen chemistries to etch very deep, high aspect ratio valleys into the silicon. Those regions protected by the metal mask remain and form the microneedles.

<100>-oriented, prime grade, 450-550 µm thick, 10-15Ω-cm silicon wafers (Nova Electronic Materials Inc., Richardson, TX) were used as the starting material. The wafers were cleaned in a solution of 5 parts by volume deionized water, 1 part 30% hydrogen peroxide, and 1 part 30% ammonium hydroxide (J.T. Baker, Phillipsburg, NJ) at approximately 80°C for 15 minutes, and then dried in an oven (Blue M Electric, Watertown, WI) at 150°C for 10 minutes. Approximately 1000 A of chromium (Mat-Vac Technology, Flagler Beach, FL) was deposited onto the wafers using a DC-sputterer (601 Sputtering System, CVC Products, Rochester, NY). The chromium layer was patterned into 20 by 20 arrays of 80 µm diameter dots with 150 µm center-to-center spacing using the lithographic process described below.

A layer of photosensitive material (1827 photoresist, Shipley, Marlborough, MA) was deposited onto the chromium layer covering the silicon wafers. A standard lithographic mask (Telic, Santa Monica, CA) bearing the appropriate dot array pattern was positioned on top of the photoresist layer. The wafer and photoresist were then exposed to ultraviolet (UV) light through the mask by means of an optical mask aligner (Hybralign Series 500, Optical Associates, Inc., Milpitas, CA). The exposed photoresist was removed by soaking the wafers in a liquid developer (354 developer, Shipley, Marlborough, MA) leaving the desired dot array of photoresist on the chromium layer. Subsequently, the wafers were dipped into a chromium etchant (CR-75; Cyanteck Fremont, CA), which etched the chromium that had been exposed during the photolithography step, leaving dot arrays of chromium (covered with photoresist) on the surface of the silicon wafer. The photoresist still present on the chromium dots formed the masks needed for fabrication of the microneedles, described below.

The microneedles were fabricated using a reactive ion etching techniques based on the Black Silicon Method developed at the University of Twente. The patterned wafers were etched in a reactive ion etcher (700 series wafer/batch Plasma Processing System, Plasma Therm, St. Petersburg, FL) with means for ensuring good thermal contact between the wafers and the underlying platen (Apiezon N, K.J. Lesker, Clairton, PA). The wafers were etched using the following gases and conditions: SF₆ (20 standard cubic centimeters per minute) and O₂ (15 standard cubic centimeters per minute) at a pressure of 150 mTorr and a power of 150 W for a run time of approximately 250 minutes. These conditions caused both deep vertical etching and slight lateral underetching. By controlling the ratio of flow rates of the SF₆ and O₂ gases used to form the plasma, the aspect ratio of the microneedles could be adjusted. The regions protected by the chromium masks remained and formed the microneedles. Etching was allowed to proceed untill the masks fell off due to underetching, resulting in an array of sharp silicon

### Comparative Example 2: Transdermal Transport Using Solid Microneedles

To determine if microfabricated microneedles could be used to enhance transdermal drug delivery, arrays of microneedles were made using a deep plasma etching technique. Their ability to penetrate human skin without breaking was tested and the resulting changes in transdermal transport were measured

Arrays of microneedles were fabricated having extremely sharp tips (radius of curvature less than 1 µm) and are ,approximately 150 µm log. Because the skin surface is not flat due to dermatoglyphics and hair, the full length of these microneedles will not penetrate the skin. All experiments were performed at room temperature (23±2°C).

The ability of the microneedles to pierce skin with without breaking was then tested. Insertion of the arrays into skin required only gentle pushing. Inspection by light and electron microscopy showed that more than 95% of microneedles within an array pierced across the stratum corneum of the epidermis samples. Moreover, essentially all of the microneedles that penetrated the epidermis remained intact. On those very few which broke, only the top 5-10 µm was damaged. Microneedle arrays could also be removed without difficulty or additional damage, as well as re-inserted into skin multiple times.

To quantitatively assess the ability of microneedles to increase transdermal transport, calcein permeability of human epidermis with and without inserted microneedle arrays was measured. Calcein crosses skin very poorly under normal circumstances and therefore represents an especially difficult compound to deliver. As expected, passive permeability of calcein across unaltered skin was very low, indicating that the epidermis samples were intact.

Insertion of microneedles into skin was capable of dramatically increasing permeability to calcein. When microneedles were inserted and left embedded in the skin, calcein permeability was increased by more than 1000-fold. Insertion of microneedles for 10 s, followed by their removal, yielded an almost 10,000-fold increase. Finally, insertion of a microneedle array for 1 h, followed by its removal, increased skin permeability by about 25,000-fold. Permeabilities for skin with microneedles inserted and then removed are higher than for skin with microneedles remaining embedded probably because the microneedles themselves or the silicon plate supporting the array may block access to the microscopic holes created in the skin. Light microscopy showed that the holes which remained in the skin after microneedles were removed were approximately 1 µm in size.

To confirm *in vitro* experiments which showed that skin permeability can be significantly increased by microneedles, studies were conducted with human volunteers. They indicated that microneedles could be easily inserted into the skin of the forearm or hand. Moreover, insertion of microneedle arrays was never reported to be painful, but sometimes elicited a mild "wearing" sensation described as a weak pressure or the feeling of a piece of tape affixed to the skin. Although transport experiments were not performed *in vivo,* skin electrical resistance was measured before and after microneedle insertion. Microneedles caused a 50-fold drop in skin resistance, a drop similar to that caused by the insertion of a 30-gauge "macroneedle." Inspection of the site immediately after microneedle insertion showed no holes visible by light microscopy. No erythema, edema, or other reaction to microneedles was observed over the hours and days which followed. This indicates that microneedle arrays can permeabilize skin in human subjects in a non-painful and safe manner.

### Example 3: Fabrication of Silicon Microtubes

Three-dimensional arrays of microtubes were fabricated from silicon, using deep reactive ion etching combined with a modified black silicon process in a conventional reactive ion etcher. The fabrication process is illustrated in Figures 5a-d. First, arrays of 40 µm diameter circular holes **32** were patterned through photoresist **34** into a 1 µm thick SiO₂ layer **36** on a two inch silicon wafer **38** (Figure 5a). The wafer **38** was then etched using deep reactive ion etching (DRIE) (Laermer, et al., "Bosch Deep Silicon Etching: Improving Uniformity and Etch Rate for Advanced MEMS Applications," Micro Electro Mechanical Systems, Orlando, Florida, USA (Jan. 17-21, 1999)). in an inductively coupled plasma (ICP) reactor to etch deep vertical holes **40.** The deep silicon etch was stopped after the holes **40** are approximately 200 µm deep into the silicon substrate **38** (Figure 5b) and the photoresist **34** was removed. A second photolithography step patterned the remaining SiO₂ layer **36** into circles concentric to the holes, thus leaving ring shaped oxide masks **34** surrounding the holes (Figure 5c). The photoresist **34** was then removed and the wafer **38** was again deep silicon etched, while simultaneously the holes **40** were etched completely through the wafer **38** (inside the SiO₂ ring) and the silicon was etched around the SiO₂ ring **38** leaving a cylinder **42** (Figure **5d****).** The resulting tubes were 150 µm in height, with an outer diameter of 80 µm, an inner diameter of 40 µm, and a tube center-to-center spacing of 300 µm.

### Example 4: Micromold Fabrication of Metal Microtubes

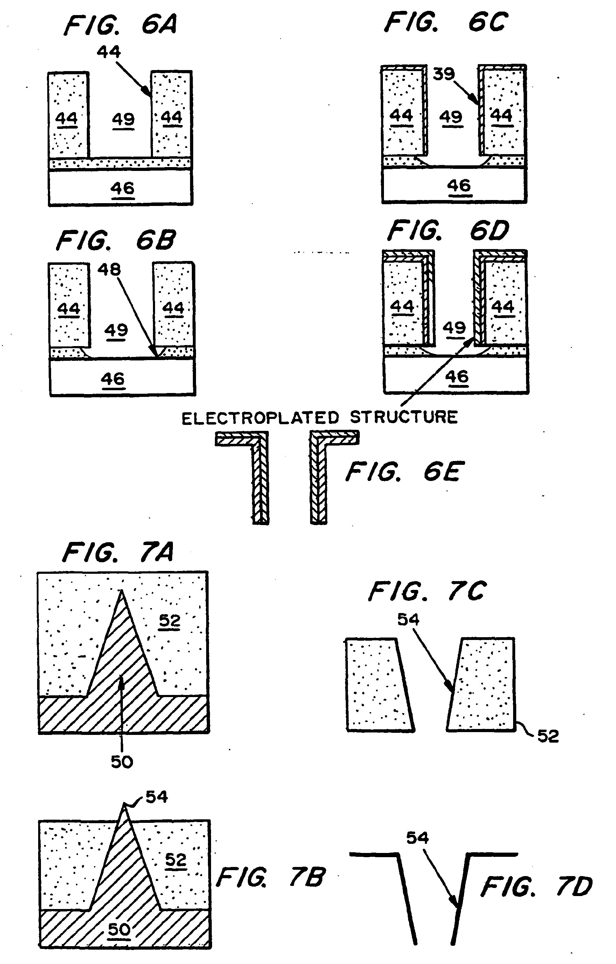

Hollow metal microtubes were prepared without dry silicon etching, using a thick, photo-defined mold of epoxy. The sequences are illustrated in Figures 6a-e. First, a thick layer of SU-8 epoxy **44** was spin cast onto a silicon or glass substrate **46** that had been coated with 30 nm of titanium **48,** the sacrificial layer. Arrays of cylindrical holes **49** were then photolithographically defined through an epoxy layer **44,** typically 150 µm thick (Figure 6a). The sacrificial layer then was partially removed using a wet etching solution containing hydrofluoric acid and water at the bottom of the cylindrical holes in the SU-8 photoresist **46** (Figure 6b). A seed layer of Ti/Cu/Ti (30 nm/200 nm/30 nm) 39 was then conformally DC sputter-deposited onto the upper surface of the epoxy mold and onto the sidewalls of the cylindrical holes **49** (Figure 6c). As shown in Figure 6c, the seed layer **39** was electrically isolated from the substrate. Subsequently, NiFe was electroplated onto the seed layer **39** (Figure 6d), the epoxy **44** was removed from the substrate, and the surrounding epoxy **44** was removed (Figure 6e). The resulting microtubes are 200 µm in height with an outer diameter of 80 µm, an inner diameter of 60 µm, and a tube center-to-center spacing of 150 µm. The holes in the interior of the microtubes protrude through the base metal supporting the tubes.

### Example 5: Micromold Fabrication of Tapered Microneedles

A micromold having tapered walls was fabricated by molding a preexisting 3-D array of microneedles, i.e. the mold-insert, and subsequently removing the mold insert. The micromold was then surface plated in a manner similar to that for the microtubes described in Example 4. The fabrication sequence is illustrated in Figures 7a-7d.

First, an array of solid silicon microneedles **50** were prepared as described in Henry, et al., "Micromachined Needles for the Transdermal Delivery of Drugs," Micro Electro Mechanical Systems, Heidelberg, Germany, Jan. 26-29, pp. 494-498 (1998). Then, a layer of epoxy **52** (SU-8) was spin cast onto the microneedle array to completely blanket the array (Figure 7a). The epoxy **52** settled during pre-bake to create a planar surface above the tips of the microneedles **50**. The epoxy **52** was then fully pre-baked, photolithographically cross-linked, and post-baked.

Then, the upper surface of the epoxy **52** was etched away using an O₂/CHF₃ plasma until approximately 1 to 2 µm of the needle tips **54** were exposed, protruding from the epoxy 52 (Figure 7b). The silicon was then selectively removed by using a SF₆ plasma (Figure 7c). The remaining epoxy mold **52** provided a negative of the microneedles with a small diameter hole where the tip of the silicon needle protruded. After the removal of the silicon, a seed layer of Ti-Cu-Ti **54** was conformally sputter-deposited onto the top and sidewalls of the epoxy micromold **52.** Following the same process sequence as described in Example 4, NiFe was then electroplated onto the seed layer **54** (Figure 7c). Finally, the epoxy was removed using an O₂/CHF₃ plasma, leaving a 20 x 20 array of NiFe hollow metal microneedles **54** (Figure 7d). The microneedles **54** were 150 µm in height with a base diameter of 80 µm, a tip diameter of 10 µm, and a needle-to-needle spacing of 150 µm.

Micromold-based microneedles also have been successfully manufactured using a process in which the epoxy mold material was replaced with PDMS. In this case, it was possible to remove the mold from the mold insert, as well as the microneedles from the mold, using only physical techniques such as peeling. This approach advantageously requires no dry etching and allows one to reuse both the mold and the mold insert.

### Example 6: Micromold Fabrication of Tapered Microneedles Using Laser-Formed Molds

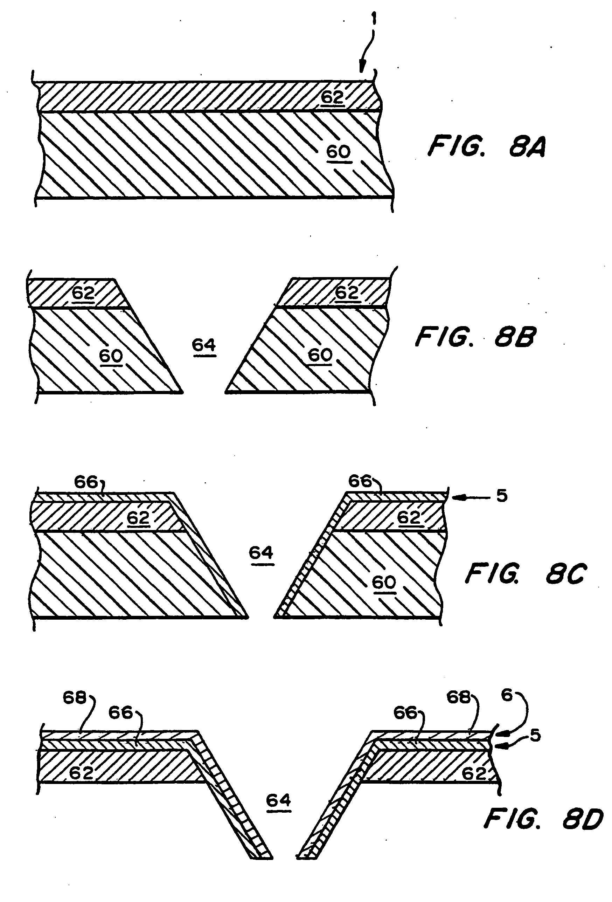

A micromold having tapered walls was fabricated by use of laser ablation techniques, as shown in Figures 8a-d. A laser-ablatable polymer sheet 60 such as KAPTON^{™} polyimide approximately 150 microns in thickness was optionally laminated to a thin (10-30 micron) metal sheet **62** such as titanium (Figure 8a). A tapered hole **64** was formed in the metal/polymer laminate **60/62** using a laser technique such as excimer laser ablation (Figure 8b). The entry hole of the laser spot was on the metal side **62,** and a through hole was made through both the metal sheet and the polymer film. The through hole **64** was tapered in combination with either defocusing or appropriate substrate motion to create a taper such that the wide end of the hole **64** (typically 40-50 microns) was on the metal side **62** and the narrow end of the hole 64 (typically 10-20 microns) was on the polymer **60** side. A thin layer of metal **66,** e.g. titanium, of thickness 0.1 micron was then deposited, e.g., using a sputter-deposition technique, in such a way that the metal **66** deposited on the metal film side and coated the polymer sidewalls, but did not coat the polymer **60** side of the laminate (Figure 8c). Electrodeposition of metal **68,** e.g., gold, to a thickness of 1 to 5 microns was then performed on the titanium-coated metal surface **66**, and polymer sidewalls curved section of **60** next to **64.** Finally, the polymer **60** was removed, using e.g. an oxygen plasma, to form the completed microneedles (Figure 8d).

Alternate polymer removal methods, such as thermal, solvent, aqueous, or photodegradation followed by solvent or aqueous removal, are also possible if the polymer material is chosen appropriately (e.g., a photoresist resin).

### Example 7: Formation of Microneedles by Embossing

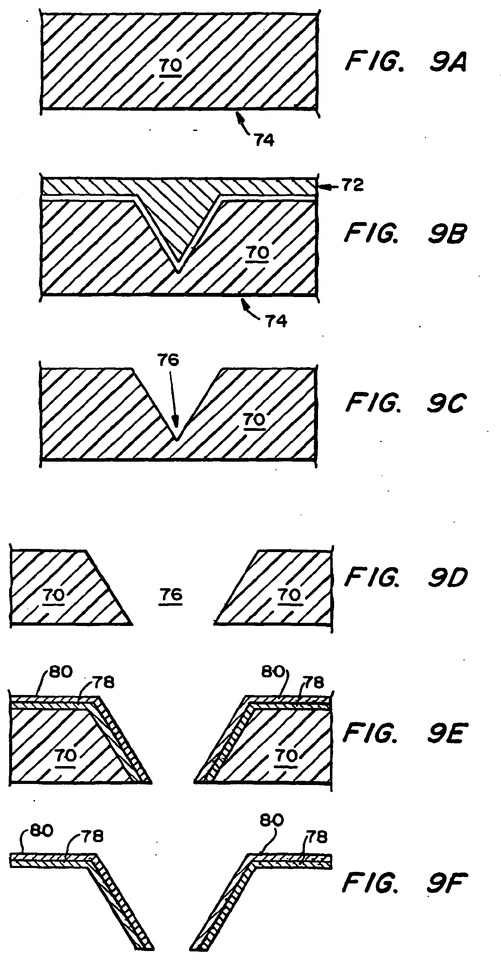

Formation of a microneedle by embossing is shown in Figures 9a-9f. A polymeric layer **70** (Figure 9a) is embossed by a solid microneedle or microneedle array **72** (Figure 9b). The array **72** is removed (Figure 9c), and the layer **70** is etched from the non-embossed side **74** until the embossed cavity **76** is exposed (Figure 9d). A metallic layer **78** is then deposited on the embossed side and the sidewalls, but not on the non-embossed side **74** (Figure 9e). This layer **78** is optionally thickened by electrodeposition of an additional metal layer **80** on top of it (Figure 9e). The polymer layer **70** is then removed to form the microneedles **78/80** (Figure 9f).

### Example 8: Transdermal Application of Hollow Microneedles

The bore of hollow microneedles must provide fluid flow with minimal clogging in order to be suitable to transport material, such as in transdermal drug delivery. Therefore, microneedles and microtubes were evaluated to determine their suitability for these functions.

Hollow metal and silicon microneedles, produced as described in Examples 3-5, were inserted through human skin epidermis with no apparent clogging of the needle bores. Scanning electron microscopy of a hollow metal (NiFe) microneedle penetrating up through the underside of human epidermis showed the microneedle remains intact, with the tip free of debris. Similarly, silicon microneedles, metal microneedles, and metal microtubes were successfully inserted through human skin. Also, the hollow microneedles were shown to permit the flow of water through their bores.

### Example 9: Drug Transport Through Microneedles Inserted Into Skin

Studies were performed with solid and hollow microneedles to demonstrate transport of molecules and fluids. As shown in Table 1, transport of a number of different compounds across skin is possible using microneedles. These studies were performed using either solid silicon microneedles or using hollow silicon microneedles made by methods described in this patent. Transport was measured across human cadaver epidermis *in vitro* using Franz diffusion chambers at 37 °C using methods described in S. Henry, D. McAllister, M.G. Allen, and M.R. Prausnitz, "Microfabricated microneedles: A novel method to increase transdermal drug delivery" J. Pharm. Sci. 87: 922-25 (1998).

The transdermal delivery of calcein, insulin, bovine serum albumin and nanoparticles was measured. Delivery refers to the ability to transport these compounds from the stratum corneum side of the epidermis to the viable epidermis side. This is the direction of transport associated with delivering drugs into the body. Removal of calcein was also measured. Removal refers to the ability to transport calcein from the viable epidermis side of the epidermis to the stratum corneum side. This is the direction of transport associated with removing from the body compounds found in the body, such as glucose.

In all cases shown in Table 1, transport of these compounds across skin occurred at levels below our detection limit when no needles were inserted into the skin. Intact skin provides an excellent barrier to transport of these compounds. In all cases examined, when solid microneedles were inserted into the skin and left in place, large skin permeabilities were measured, indicating that the microneedles had created pathways for transport across the skin. Furthermore, in all cases, when solid microneedles were inserted into the skin and then removed, even greater skin permeabilities resulted. Finally, when hollow microneedles were inserted into the skin and left in place, still greater skin permeabilities resulted for those compounds tested. These studies show that microneedles can dramatically increase skin permeability and can thereby increase transport of a number of different compounds across the skin. It also shows that when solid microneedles are used, a preferred embodiment involves inserting and then removing microneedles, rather than leaving them in place. It also shows that using hollow microneedles are a preferred embodiment over the use of solid microneedles.

In Table 2, the flow rate of water through hollow silicon microneedles is shown as a function of applied pressure. These data demonstrate that significant flow rates of water through microneedles can be achieved at modest pressures.

**TABLE1: Transport of Drugs Through Microneedles Inserted Into Skin**

| Compound | No needles | Solid needles inserted | Solid needles inserted and removed | Hollow needles inserted |
|---|---|---|---|---|
| Calcein delivery | ** | 4 x 10⁻³ | 1 x 10⁻² | 1 x 10⁻¹ |
| Calcein removal | ** | 2x10⁻³ | 1x10⁻² | n.a. |
| Insulin delivery | ** | 1 x 10⁻⁴ | 1 x 10⁻² | n.a. |
| Bovine serum albumin delivery | ** | 9 x 10⁻⁴ | 8 x 10⁻³ | 9x10⁻² |
| Nanoparticle delivery | ** | n.a. | 3 x 10⁻⁵ | n.a. |

| | | | | |
|---|---|---|---|---|
| ** means that the transport was below the detection limit. n.a. means that the data are not available. Nanoparticles were made of latex with a diameter of approximately 100 nm. | | | | |

**TABLE 2: Flow Rate of Water Through Hollow Silicon Microneedles as a Function of Applied Pressure**

| Pressure (psi) | Flow rate (ml/min) |
|---|---|
| 1.0 | 16 |
| 1.5 | 24 |
| 2.0 | 31 |
| 2.5 | 38 |
| 3.0 | 45 |

## Claims

1. A device (10) for transport of molecules or energy across biological barriers comprising
a plurality of hollow or porous microneedles (12) having a length between 10 µm and 1 mm, wherein at least a portion of the shaft of the microneedles (12) has a width between about 1 µm and 200 µm, and
a substrate (11) to which the microneedles (12) are attached or integrally formed, wherein the microneedles (12) extend at an angle from a surface of the substrate.

2. The device (10) of claim 1 wherein the angle is about 90°.

3. The device (10) of claim 1 which is formed using a microfabricated mold.

4. The device (10) of any of claims 1-3 wherein the microneedles (12) are hollow.

5. The device (10) of any of claims 1-4 wherein the microneedles (12) are formed by a method comprising the steps:
(a) forming a micromold having sidewalls which define a surface of the microneedles (12);
(b) depositing material on sidewalls to form the hollow microneedles (12); and
(c) removing the micromold from the microneedles (12).

6. The device (10) of claim 5 further comprising forming the mold using a laser to selectively remove material.

7. The device (10) of claim 1 wherein the microneedles (12) are fabricated using a micromachining technique selected from the group consisting of lithography, plasma etching, wet chemical etching, dry etching, thermal oxidation of silicon, electroplating, electroless plating, boron diffusion, phosphorus diffusion, arsenic diffusion, antimony diffusion, ion implantation, film deposition, sputtering, chemical vapor deposition, epitaxy, chemical anodization, electrochemical anodization, micromolding, laser ablation, and combinations thereof.

8. The device (10) of any of claims 1-7 wherein the length of the microneedles (12) is between about 30 µm and 200 µm.

9. The device (10) of any of claims 1-8 wherein the microneedles (12) have an outer diameter between about 10 µm and about 100 µm.

10. The device (10) of claim 1 or 4 wherein the hollow microneedles (12) have an inner diameter between about 3 µm and about 80 µm.

11. The device (10) of claim 1 wherein the microneedles (12) are hollow and each comprise a base end which tapers to a sharp tip end, and wherein the length of the microneedles (12) is between about 30 µm and 200 µm.

12. The device (10) of any of claims 1-11 wherein the microneedles (12) are formed of a material selected from the group consisting of silicon, silicon dioxide, metals, ceramics, polymers, and combinations thereof.

13. The device (10) of claim 12 wherein the microneedles (12) are formed of a metal.

14. The device (10) of claim 12 wherein the metal is selected from the group consisting of nickel, iron, gold, titanium, tin, copper, stainless steel, platinum, palladiums and alloys thereof.

15. The device (10) of claim 12 wherein the material is a biodegradable polymer selected from the group consisting of poly(hydroxy acid)s, polyanhydrides, poly(ortho)esters, polyurethanes, poly(butyric acid)s, poly(valeric acid)s, and poly(lactide-co-caprolactone)s.

16. The device (10) of claim 1 wherein the microneedles (12) are microtubes.

17. The device (10) of claim 1 wherein the microneedles (12) each comprise a shaft having a circular or non-circular cross-sectional area perpendicular to the axis of the microneedle.

18. The device (10) of claim 1 wherein the microneedles (12) each have a configured or grooved outer surface.

19. The device (10) of any of claims 1-18 wherein the molecules or energy is delivered from one or more chambers or reservoirs in connection with at least one of the microneedles (12).

20. The device (10) of claim 19 further comprising a means for controlling the flow of molecules or energy through the microneedles (12).

21. The device (10) of claim 20 wherein the means is selected from the group consisting of permeable membranes, fracturable impermeable membranes, gates, valves, and pumps.

22. The device (10) of any of claims 1-21 further comprising a means for temporarily securing the microneedle device (10) to the biological barrier.

23. The device (10) of claim 22 wherein the securing means is selecoed from the group consisting of collars, tabs, adhesive agents, and combinations thereof.

24. The device (10) of claim 1 wherein the device is electrochemically, thermally, mechanically or magnetically active.

25. The device (10) of claim 1 wherein the surface of the microneedles (12) is shaped or comprises a material to facilitate (i) passage of the microneedles (12) across the biological barrier or (ii) passage of the molecules across the biological barrier by means of the microneedles (12).

26. The device (10) of claim 1 wherein the microneedles (12) form a mechanical support when inserted into a tissue.

27. The device (10) of claim 26 wherein the mechanical support forms a vascular or urethral stent.

28. The device (10) of claim 1 further comprising a flexible backing.

29. The device (10) of any of claims 1-28 further comprising molecules to be released or delivered

30. The device (10) of claim 29 wherein the molecules are incorporated into and released from the microneedles (12) after the microneedles (12) are administered.

31. The device (10) of claim 30 wherein the microneedles (12) are formed of a biodegradable material and sheared off at a site of administration.

32. A method of making the microneedle device (10) of any of claims 1-31, the method comprising:
forming a microneedle (12) using a micromachining technique selected from the group consisting of lithography, plasma etching, wet chemical etching, dry etching, thermal oxidation of silicon, electroplating, electroless plating, boron diffusion, phosphorus diffusion, arsenic diffusion, antimony diffusion, ion implantation, film deposition, sputtering, chemical vapor deposition, epitaxy, chemical anodization, electrochemical anodization, micromolding, laser ablation, and combinations thereof.

33. A method for making a device (10) according to any one of claims 1 to 31 having a plurality of hollow microneedles (12), and a substrate to which the microneedles (12) are attached or integrally formed, wherein the microneedles (12) extend at an angle from a surface of the substrate, the method comprising
forming the microneedles (12) using a micromold having sidewalls which define a surface of the microneedle.

34. The method of claim 33 wherein one or more holes are photolithographically defined in a second substrate, thereby forming the micromold.

35. The method of claim 33 further comprising applying a metal, or other material having different properties than the material forming the mold, to the sidewalls to form the hollow microneedles (12) and then removing the micromold from the microneedle (12).

36. The method of claim 33 further comprising filling the micromold with a fluid material that is hardened in the mold to form the microneedle.

37. The method of claim 36 which utilizes injection molding or reaction injection molding.

38. The method of claim 36 wherein the micromold is fabricated by forming a mold from a mold-insert.

39. The method of claim 38 wherein the mold or the mold-insert is formed from an epoxy or a polydimethylsiloxane.

40. The method of claim 38 or 39 wherein the mold insert is an array of microneedles (12).

41. The method of claim 40 for forming hollow microneedles (12), comprising the steps of
(a) layering a removable material onto the array to cover the miconeedles (12) of the mold-insert,
(b) removing a part of the layer of removable material to expose the tips the microneedles (12) of the mold-insert, and
(c) removing the mold-insert to yield a micromold.

42. The method of claim 41 further comprising
(d) applying a metal, or other material having properties distinct from the material forming the mold, onto the micromold to form the microneedles, and
(e) removing the micromold from the microneedles (12).

43. The method of claim 33 wherein the the micromold is shaped by embossing.

44. The method of claim 33 wherein the micromold is shaped using a laser to selectively remove material.

45. A method for making hollow microneedles or microtubes (12) as defined in any one of claims 1 to 31 comprising
(a) forming a mask on a substrate,
(b) selectively removing the substrate to form the microneedle (12) or microtube shape, and
(c) making a hollow bore in the microneedle or microtube shape.

46. The method of claim 45 wherein the bore is made prior to forming the outer walls of the microneedle or microtube (12).

47. The method of claim 45 wherein the bore is made after forming the microneedle or microtube shape (12).

48. The method of claim 45 for forming microneedles wherein the microneedle shape is formed by tapered outer walls of the substrate.

49. The method of claim 44 wherein tapered hole are formed by moving the laser relative to the micromold.

## Patentansprüche

1. Eine Vorrichtung (10) für den Transport von Molekülen oder Energie über biologische Barrieren umfassend,
eine Vielzahl hohler oder poröser Mikronadeln (12),
welche eine Länge zwischen 10 µm und 1 mm haben, worin mindestens ein Teil des Schaftes der Mikronadeln (12) eine Breite zwischen etwa 1 µm und 200 µm hat, und
ein Substrat (11), an das die Mikronadeln (12) angelagert oder ganzheitlich geformt sind, worin die Mikronadeln (12) sich in einem Winkel von einer Oberfläche des Substrates erstrecken.

2. Die Vorrichtung (10) nach Anspruch 1, worin der Winkel etwa 90° ist.

3. Die Vorrichtung (10) nach Anspruch 1, welche unter Verwendung einer mikrofabrizierten Form hergestellt ist.

4. Die Vorrichtung (10) nach einem der Ansprüche 1 bis 3, worin die Mikronadeln (12) hohl sind.

5. Die Vorrichtung (10) nach einem der Ansprüche 1 bis 4, worin die Mikronadeln (12) nach einem Verfahren hergestellt werden, das folgende Schritte umfasst:
(a) Bilden einer Mikroform, welche Seitenwände hat, die eine Oberfläche der Mikronadeln (12) definieren;
(b) Auftragen des Materials auf die Seitenwände, um die hohlen Mikronadeln (12) zu bilden; und
(c) Entfernen der Mikroform von den Mikronadeln (12).

6. Die Vorrichtung (10) nach Anspruch 5, ferner umfassend die Herstellung der Form unter Verwendung eines Lasers, um selektiv Material zu entfernen.

7. Die Vorrichtung (10) nach Anspruch 1, worin die Mikronadeln (12) mit einer Mikromaschinentechnik hergestellt werden, welche aus der Gruppe bestehend aus Lithographie, Plasmaätzen, nasschemischer Strukturätzung, Trockenätzung, thermischer Oxidation von Silizium, Galvanisierung, stromloser Plattierung, Bordiffusion, Phosphordiffusion, arsenhaltiger Diffusion, Antimondiffusion, lonenimplantation, Dünnschichtniederschlagung, Zerstäubungsablagerungsverfahren, chemischer Dampfphasenabscheidung, Epitaxie, chemischer Eloxierung, Mikroabformung, Laserablation und Kombinationen hiervon, ausgewählt wird.

8. Die Vorrichtung (10) nach einem der Ansprüche 1 bis 7, worin die Länge der Mikronadeln (12) zwischen etwa 30 µm und 200 µm ist.

9. Die Vorrichtung (10) nach einem der Ansprüche 1 bis 8, worin die Mikronadeln (12) einen Außendurchmesser zwischen etwa 10 µm und etwa 100 µm haben.

10. Die Vorrichtung (10) nach einem der Ansprüche 1 bis 4, worin die hohlen Mikronadeln (12) einen Innendurchmesser zwischen etwa 3 µm und etwa 80 µm haben.

11. Die Vorrichtung (10) nach Anspruch 1, worin die Mikronadeln (12) hohl sind und jeweils ein niedriges Ende enthalten, das sich zu einem scharfen Spitzenende zuspitzt, worin die Länge der Mikronadeln (12) zwischen etwa 30 µm und etwa 200 µm ist.

12. Die Vorrichtung (10) nach einem der Ansprüche 1 bis 11, worin die Mikronadeln (12) aus einem Material hergestellt sind, welches aus der Gruppe, die Silizium, Siliciumdioxid, Metallen, Keramiken, Polymeren und Kombinationen hiervon umfasst, ausgewählt ist.

13. Die Vorrichtung (10) nach Anspruch 12, worin die Mikronadeln (12) aus Metall hergestellt sind.

14. Die Vorrichtung (10) nach Anspruch 12, worin das Metall aus der Gruppe, Nickel, Eisen, Gold, Titan, Zinn, Kupfer, rostfreien Stahl, Platin, Palladium und Legierungen hiervon umfasst, ausgewählt ist.

15. Die Vorrichtung (10) nach Anspruch 12, worin das Material ein biologisch abbaubares Polymer ist, das aus der Gruppe, die aus Polyhydroxysäure/n, Polyanhydride, Polyorthoester, Polyurethan, Polybuttersäure/n, Polyvaleriansäure/n und Polylactid-Co-Caprolacton/e besteht, ausgewählt ist.

16. Die Vorrichtung (10) nach Anspruch 1, worin die Mikronadeln (12) Mikroröhren sind.

17. Die Vorrichtung (10) nach Anspruch 1, worin die Mikronadeln (12) einen Schaft umfassen, welcher eine kreisförmige oder nichtkreisförmige Querschnittsfläche senkrecht zur Achse der Mikronadel hat.

18. Die Vorrichtung (10) nach Anspruch 1, worin die Mikronadeln (12) jeweils eine konfigurierte oder gerillte Außenfläche haben.

19. Die Vorrichtung (10) nach einem der Ansprüche 1 bis 18, worin die Moleküle oder die Energie von einem oder mehreren Kammern oder Vorratsbehältern, welche in Verbindung mit mindestens einer der Mikronadeln (12) stehen, geliefert werden/wird.

20. Die Vorrichtung (10) nach Anspruch 19, ferner umfassend Mittel für die Steuerung des Flusses der Moleküle oder der Energie durch die Mikronadeln (12).

21. Die Vorrichtung (10) nach Anspruch 20, worin die Mittel aus einer Gruppe von permeablen Membranen, brüchigen undurchlässigen Membranen, Gattern, Ventilen und Pumpen ausgewählt ist.

22. Die Vorrichtung (10) nach einem der Ansprüche 1 bis 21, ferner umfassend Mittel für die vorübergehende Befestigung der Mikronadelvorrichtung (10) an der biologischen Barriere.

23. Die Vorrichtung (10) nach Anspruch 22, worin die Befestigungsmittel aus einer Gruppe bestehend aus Manschetten, Laschen, Haftmitteln und Kombinationen hiervon ausgewählt sind.

24. Die Vorrichtung (10) nach Anspruch 1, worin die Vorrichtung elektrochemisch, thermisch, mechanisch oder magnetisch aktiv ist.

25. Die Vorrichtung (10) nach Anspruch 1, worin die Oberfläche der Mikronadeln (12) gestaltet ist oder ein Material umfasst, um (i) die Passage der Mikronadeln (12) durch die biologische Barriere oder (ii) die Passage der Moleküle durch die biologische Barriere mittels der Mikronadeln (12) zu vereinfachen.

26. Die Vorrichtung (10) nach Anspruch 1, worin die Mikronadeln (12) einen mechanischen Träger ausbilden, wenn sie in das Gewebe eingebracht werden.

27. Die Vorrichtung (10) nach Anspruch 26, worin der mechanische Träger einen vaskulären oder urethralen Stent ausbildet.

28. Die Vorrichtung (10) nach Anspruch 1, ferner umfassend einen flexiblen Schutzträger.

29. Die Vorrichtung (10) nach einem der Ansprüche 1 bis 28, welches ferner freizusetzende oder abzugebende Moleküle umfasst.

30. Die Vorrichtung (10) nach Anspruch 29, worin die Moleküle eingebaut werden in und freigesetzt werden aus den Mikronadeln (12), nachdem die Mikronadeln (12) appliziert wurden.

31. Die Vorrichtung (10) nach Anspruch 30, worin die Mikronadeln (12) aus einem biologisch abbaubaren Material gebildet und an einer Applikationsstelle abgeschert werden.

32. Ein Verfahren zur Herstellung der Mikronadelvorrichtung (10) nach einem der Ansprüche 1 bis 31, wobei das Verfahren umfasst:
Herstellen der Mikronadeln (12) mit einer Mikromaschinentechnik, welche aus der Gruppe bestehend aus Lithographie, Plasmaätzen, nass chemischer Strukturätzung, Trockenätzung, thermischer Oxidation von Silizium, Galvanisierung, stromloser Plattierung, Bordiffusion, Phosphordiffusion, arsenhaltiger Diffusion, Antimondiffusion, Ionenimplantation, Dünnschichtniederschlagung, Zerstäubungsablagerungsverfahren, chemischer Dampfphasenabscheidung, Epitaxie, chemischer Eloxierung, Mikroabformung, Laserablation und Kombinationen hiervon ausgewählt ist.

33. Ein Verfahren zur Herstellung einer Vorrichtung (10) nach einem der Ansprüche 1 bis 31, mit einer Vielzahl hohler Mikronadeln (12) und einem Substrat, an das die Mikronadeln (12) angelagert oder ganzheitlich geformt sind, worin die Mikronadeln (12) sich in einem Winkel von einer Oberfläche des Substrates erstrecken, wobei das Verfahren umfasst
Bilden der Mikronadeln (12) unter Verwendung einer Mikroform, welche Seitenwände hat, die eine Oberfläche der Mikronadeln (12) definieren.

34. Das Verfahren nach Anspruch 33, worin ein oder mehrere Löcher photolithographisch in einem zweiten Substrat definiert werden, um dadurch die Mikroform zu bilden.

35. Das Verfahren nach Anspruch 33, welches ferner das Applizieren eines Metalls oder sonstigen Materials auf die Seitenwände umfasst, das andere Eigenschaften hat als das Material, welches die Form bildet, um die hohlen Mikronadeln (12) zu bilden und dann die Mikroform von der Mikronadel (12) zu entfernen.

36. Das Verfahren nach Anspruch 33, welches ferner umfasst, dass die Mikroform mit einem flüssigen Material gefüllt wird, das in der Form gehärtet wird, um die Mikronadel zu bilden.

37. Das Verfahren nach Anspruch 36, welches Spritzgusstechnik oder Reaktionsspritzgusstechnik verwendet.

38. Das Verfahren nach Anspruch 36, worin die Mikroform durch Bilden einer Form aus einem Formeinsatz hergestellt wird.

39. Das Verfahren nach Anspruch 38, worin die Form oder der Formeinsatz aus einem Epoxid oder einem Polydimethylsiloxan hergestellt wird.

40. Das Verfahren nach Anspruch 38 oder 39, worin der Formeinsatz eine Anordnung aus Mikronadeln (12) ist.

41. Das Verfahren nach Anspruch 40 zur Herstellung von hohlen Mikronadeln (12), welches folgende Schritte umfasst
(a) Schichten eines entfernbaren Materials auf die Anordnung zur Abdeckung der Mikronadeln (12) des Formeinsatzes,
(b) Entfernen eines Teils der Schicht des entfernbaren Materials zur Freilegung der Spitzen der Mikronadeln (12) des Formeinsatzes und
(c) das Entfernen des Formeinsatzes zur Gewinnung einer Mikroform.

42. Das Verfahren nach Anspruch 41, welches ferner
(d) das Applizieren eines Metalls oder eines anderen Materials, welches andere Eigenschaften besitzt als das Material, welches die Form bildet, auf die Mikroform zur Herstellung der Mikronadeln (12) und
(e) das Entfernen der Mikroform von den Mikronadeln (12)
umfasst.

43. Das Verfahren nach Anspruch 33, worin die Mikroform durch Prägung geformt wird.

44. Das Verfahren nach Anspruch 33, worin die Mikroform unter Verwendung eines Lasers zur selektiven Entfernung des Materials geformt wird.

45. Ein Verfahren zur Herstellung hohler Mikronadeln (12) oder Mikroröhren wie in einem der Ansprüche 1 bis 31 definiert, bestehend aus
(a) Herstellen einer Maske auf einem Substrat,
(b) Selektives Entfernen des Substrates zur Formung der Mikronadel- (12) oder Mikroröhrenform und
(c) Herstellen einer hohlen Bohrung in der Mikronadel- oder Mikroröhrenform.

46. Das Verfahren nach Anspruch 45, worin die Bohrung vor der Formung der Außenwände der Mikronadeln oder Mikroröhre (12) gemacht wird.

47. Das Verfahren nach Anspruch 45, worin die Bohrung nach Herstellung der Mikronadel- (12) oder Mikroröhrenform gemacht wird.

48. Das Verfahren nach Anspruch 45 zur Herstellung von Mikronadeln (12), worin die Mikronadelform durch konische Außenwände des Substrats gebildet wird.

49. Das Verfahren nach Anspruch 44, worin konische Löcher durch Bewegung des Lasers relativ zur Mikroform geformt werden.

## Revendications

1. Dispositif (10) pour le transport de molécules ou d'énergie à travers des barrières biologiques comprenant
une pluralité de microaiguilles creuses ou poreuses (12) ayant une longueur comprise entre 10 µm et 1 mm, où au moins une partie de la tige des microaiguilles (12) a une largeur comprise entre environ 1 µm et 200 µm, et
un substrat (11) auquel les microaiguilles (12) sont attachées ou formées solidairement, où les microaiguilles (12) s'étendent selon un angle depuis une surface du substrat.

2. Dispositif (10) selon la revendication 1, dans lequel l'angle est d'environ 90

3. Dispositif (10) selon la revendication 1, qui est formé en utilisant un moule microfabriqué.

4. Dispositif (10) selon l'une quelconque des revendications 1 à 3, dans lequel les microaiguilles (12) sont creuses.

5. Dispositif (10) selon l'une quelconque des revendications 1 à 4, dans lequel les microaiguilles (12) sont formées par un procédé comprenant les étapes consistant à :
(a) former un micromoule comportant des parois latérales qui définissent une surface des microaiguilles (12) ;
(b) déposer un matériau sur les parois latérales pour former les microaiguilles creuses (12) et
(c) enlever le micromoule des microaiguilles (12).

6. Dispositif (10) selon la revendication 5, comprenant en outre la formation du moule en utilisant un laser pour enlever sélectivement le matériau.

7. Dispositif (10) selon la revendication 1, dans lequel les microaiguilles (12) sont fabriquées en utilisant une technique de micro-usinage choisie dans le groupe consistant en lithographie, gravure plasma, gravure chimique humide, gravure sèche, oxydation thermique de silicium, électroplaquage, plaquage anélectrolytique, diffusion de bore, diffusion de phosphore, diffusion d'arsenic, diffusion d'antimoine, implantation ionique, dépôt de film, pulvérisation cathodique, dépôt chimique en phase vapeur, épitaxie, anodisation chimique, anodisation électrochimique, micromoulage, ablation laser et leurs combinaisons.

8. Dispositif (10) selon l'une quelconque des revendications 1 à 7, dans lequel la longueur des microaiguilles (12) est comprise entre environ 30 µm et 200 µm.

9. Dispositif (10) selon l'une quelconque des revendications 1 à 8, dans lequel les microaiguilles (12) ont un diamètre externe compris entre environ 10 µm et environ 100 µm.

10. Dispositif (10) selon la revendication 1 ou 4, dans lequel les microaiguilles creuses (12) ont un diamètre interne compris entre environ 3 µm et environ 80 µm.

11. Dispositif (10) selon la revendication 1, dans lequel les microaiguilles (12) sont creuses et comprennent chacune une extrémité de base qui s'effile vers une extrémité d'embout vif, et où la longueur des microaiguilles (12) est comprise entre environ 30 µm et 200 µm.

12. Dispositif (10) selon l'une quelconque des revendications 1 à 11, dans lequel les microaiguilles (12) sont formées d'un matériau choisi dans le groupe consistant en le silicium, le dioxyde de silicium, les métaux, les céramiques, les polymères et leurs combinaisons.

13. Dispositif (10) selon la revendication 12, dans lequel les microaiguilles (12) sont formées d'un métal.

14. Dispositif (10) selon la revendication 12, dans lequel le métal est choisi dans le groupe consistant en le nickel, le fer, l'or, le titane, l'étain, le cuivre, l'acier inoxydable, le platine, le palladium et leurs alliages.

15. Dispositif (10) selon la revendication 12, dans lequel le matériau est un polymère biodégradable choisi dans le groupe consistant en les poly(hydroxy acides), les poly(anhydrides), les poly(ortho esters), les poly(uréthanes), les poly(acides butyriques), les poly(acides valériques) et les poly(lactide-co-caprolactones).

16. Dispositif (10) selon la revendication 1, dans lequel les microaiguilles (12) sont des microtubes.

17. Dispositif (10) selon la revendication 1, dans lequel les microaiguilles (12) comprennent chacune une tige ayant une aire en coupe circulaire ou non circulaire perpendiculaire à l'axe de la microaiguille.

18. Dispositif (10) selon la revendication 1, dans lequel les microaiguilles (12) ont chacune une surface externe configurée ou rainurée.

19. Dispositif (10) selon l'une quelconque des revendications 1 à 18, dans lequel les molécules ou l'énergie sont délivrées depuis une ou plusieurs chambres ou réservoirs raccordés avec au moins une des microaiguilles (12).

20. Dispositif (10) selon la revendication 19, comprenant en outre un moyen pour réguler l'écoulement des molécules ou de l'énergie à travers les microaiguilles (12).

21. Dispositif (10) selon la revendication 20, dans lequel le moyen est choisi dans le groupe consistant en les membranes perméables, les membranes imperméables fracturables, les grilles, les soupapes et les pompes.

22. Dispositif (10) selon l'une quelconque des revendications 1 à 21, comprenant en outre un moyen pour fixer temporairement le dispositif à microaiguilles (10) à la barrière biologique.

23. Dispositif (10) selon la revendication 22, dans lequel le moyen de fixation est choisi dans le groupe consistant en les colliers, les pattes, les agents adhésifs et leurs combinaisons.

24. Dispositif (10) selon la revendication 1, dans lequel le dispositif est électrochimiquement, thermiquement, mécaniquement ou magnétiquement actif.

25. Dispositif (10) selon la revendication 1, dans lequel la surface des microaiguilles (12) est façonnée ou comprend un matériau pour faciliter (i) le passage des microaiguilles (12) à travers la barrière biologique ou (ii) le passage des molécules à travers la barrière biologique au moyen des microaiguilles (12).

26. Dispositif (10) selon la revendication 1, dans lequel les microaiguilles (12) forment un support mécanique, une fois insérées dans un tissu.

27. Dispositif (10) selon la revendication 26, dans lequel le support mécanique forme un stent vasculaire ou urétral.

28. Dispositif (10) selon la revendication 1, comprenant en outre un renforcement flexible.

29. Dispositif (10) selon l'une quelconque des revendications 1 à 28, comprenant en outre des molécules à libérer ou à délivrer.

30. Dispositif (10) selon la revendication 29, dans lequel les molécules sont incorporées dans et libérées depuis les microaiguilles (12) après administration des microaiguilles (12).

31. Dispositif (10) selon la revendication 30, dans lequel les microaiguilles (12) sont formées d'un matériau biodégradable, et détachées au niveau d'un site d'administration.

32. Procédé de fabrication du dispositif à microaiguilles (10) selon l'une quelconque des revendications 1 à 31, le procédé comprenant l'étape consistant à :
former une microaiguille (12) en utilisant une technique de micro-usinage choisie dans le groupe consistant en lithographie, gravure plasma, gravure chimique humide, gravure sèche, oxydation thermique de silicium, électroplaquage, plaquage anélectrolytique, diffusion de bore, diffusion de phosphore, diffusion d'arsenic, diffusion d'antimoine, implantation ionique, dépôt de film, pulvérisation cathodique, dépôt chimique en phase vapeur, épitaxie, anodisation chimique, anodisation électrochimique, micromoulage, ablation laser et leurs combinaisons.

33. Procédé de fabrication d'un dispositif (10) selon l'une quelconque des revendications 1 à 31, comportant une pluralité de microaiguilles creuses (12) et un substrat auquel les microaiguilles (12) sont attachées ou formées solidairement, où les microaiguilles (12) s'étendent selon un angle depuis une surface du substrat, le procédé comprenant l'étape consistant à
former les microaiguilles (12) en utilisant un micromoule ayant des parois latérales qui définissent une surface de la microaiguille.

34. Procédé selon la revendication 33, dans lequel un ou plusieurs trous sont définis photolithographiquement dans un second substrat, formant ainsi le micromoule.

35. Procédé selon la revendication 33, comprenant en outre une application d'un métal, ou d'un autre matériau ayant des propriétés différentes du matériau formant le moule, aux parois latérales pour former les microaiguilles creuses (12) puis l'enlèvement du micromoule des microaiguilles (12).

36. Procédé selon la revendication 33, comprenant en outre le remplissage du micromoule avec un matériau fluide qui est durci dans le moule pour former la microaiguille.

37. Procédé selon la revendication 36, qui utilise un moulage par injection ou un moulage par injection à réaction.

38. Procédé selon la revendication 36, dans lequel le micromoule est fabriqué en formant un moule à partir d'un insert de moule.

39. Procédé selon la revendication 38, dans lequel le moule ou l'insert de moule est formé d'un époxy ou d'un poly(diméthylsiloxane).

40. Procédé selon la revendication 38 ou 39, dans lequel l'insert de moule est un réseau de microaiguilles (12).

41. Procédé selon la revendication 40 pour former des microaiguilles creuses (12), comprenant les étapes consistant à
(a) former en couches un matériau enlevable sur le réseau pour couvrir les microaiguilles (12) de l'insert de moule,
(b) enlever une partie de la couche du matériau enlevable pour exposer les embouts des microaiguilles (12) de l'insert de moule, et
(c) enlever l'insert de moule pour donner un micromoule.

42. Procédé selon la revendication 41, comprenant en outre l'étape consistant à
(d) appliquer un métal, ou un autre matériau ayant des propriétés distinctes du matériau formant le moule, sur le micromoule pour former les microaiguilles, et
(e) enlever le micromoule des microaiguilles (12).

43. Procédé selon la revendication 33, dans lequel le micromoule est façonné par bosselage.

44. Procédé selon la revendication 33, dans lequel le micromoule est façonné en utilisant un laser pour enlever sélectivement le matériau.

45. Procédé de fabrication de microaiguilles creuses ou microtubes (12) tel que défini dans l'une quelconque des revendications 1 à 31, comprenant les étapes consistant à
(a) former un masque sur un substrat,
(b) enlever sélectivement le substrat pour former la forme de microaiguille (12) ou de microtube, et
(c) faire un alésage creux dans la forme de microaiguille ou de microtube.

46. Procédé selon la revendication 45, dans lequel l'alésage est fait avant de former les parois externes de la microaiguille ou du microtube (12).

47. Procédé selon la revendication 45, dans lequel l'alésage est fait après formation de la forme de microaiguille ou de microtube (12).

48. Procédé selon la revendication 45 pour former des microaiguilles, dans lequel la forme de microaiguille est formée par des parois externes effilées du substrat.

49. Procédé selon la revendication 44., dans lequel des trous effilés sont formés en déplaçant le laser par rapport au micromoule.
